(19) Europäisches Patentamt
European Patent Office
Office européen des brevets

(11) EP 1 313 444 B1

(12) **EUROPÄISCHE PATENTSCHRIFT**

(45) Veröffentlichungstag und Bekanntmachung des Hinweises auf die Patenterteilung:
**17.11.2004 Patentblatt 2004/47**

(21) Anmeldenummer: **01976085.9**

(22) Anmeldetag: **16.08.2001**

(51) Int Cl.⁷: **A61K 7/48**

(86) Internationale Anmeldenummer:
**PCT/EP2001/009438**

(87) Internationale Veröffentlichungsnummer:
**WO 2002/015865 (28.02.2002 Gazette 2002/09)**

(54) **HAUTKOSMETISCHE FORMULIERUNGEN MIT COPOLYMEREN AUS VINYLCARBOXYLATEN, POLYETHERHALTIGEN VERBINDUNGEN UND WEITEREN MONOMEREN**

SKIN COSMETIC FORMULATIONS COMPRISING COPOLYMERS FROM VINYL CARBOXYLATES, POLYETHER COMPOUNDS AND FURTHER MONOMERS

FORMULATIONS COSMETIQUES POUR LA PEAU CONTENANT DES COPOLYMERES DE CARBOXYLATES DE VINYLE, DES COMPOSANTS POLYETHER ET DES MONOMERES ADDITIONELS

(84) Benannte Vertragsstaaten:
**DE FR GB**

(30) Priorität: **22.08.2000 DE 10041220**

(43) Veröffentlichungstag der Anmeldung:
**28.05.2003 Patentblatt 2003/22**

(73) Patentinhaber: **BASF AKTIENGESELLSCHAFT**
**67056 Ludwigshafen (DE)**

(72) Erfinder:
- **GOTSCHE, Michael**
  **68167 Mannheim (DE)**
- **WOOD, Claudia**
  **69469 Weinheim (DE)**
- **DIEING, Reinhold**
  **67346 Speyer (DE)**
- **JENTZSCH, Axel**
  **68167 Mannheim (DE)**

(56) Entgegenhaltungen:
**US-A- 4 240 450**
**US-A- 4 796 646**
**US-A- 4 876 083**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 313 444 B1

## Beschreibung

Die vorliegende Erfindung betrifft wässrige oder wässrig/alkoholische hautkosmetische Formulierungen enthaltend als Filmbildner Polymerisate, hergestellt durch Polymerisation von Vinylestern und optional weiteren radikalisch copolymerisierbaren Monomeren in Gegenwart einer polyetherhaltigen Verbindung.

Pfropfpolymerisate von Polyvinylalkohol auf Polyalkylenglykole sind bereits bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

DE 199 07587.5 beschreibt die Verwendung von Pfropfpolymerisaten von Vinylestern auf Polyether für die Haarkosmetik.

An hautkosmetische Formulierungen werden eine Reihe von Anforderungen gestellt:

sie sollten wasserfest bzw. wasserbeständig sein,
sie sollten möglichst wenig auf Textilien und Kleidung übertragbar sein,
sie sollten sich auf der damit behandelten Körperregion (Haut, Wimpern, Lippen, Augenbrauen, Augenlider)gut verteilen,
sie sollten feuchtigkeitsbindend wirken,
sie sollten ein angenehmes Hautgefühl vermitteln,
sie sollten keine Klebrigkeit nach der Anwendung aufweisen.

Ziel der Erfindung war es daher, hautkosmetische Formulierungen mit filmbildenden Polymeren bereitzustellen, welche mindestens eine der o.g. Anforderungen in besonders guter Weise erfüllen und damit eine verbesserte Hautpflege ermöglichen.

Die Aufgabe wurde erfindungsgemäß gelöst durch Verwendung von Polymerisaten, die erhältlich sind durch radikalische Polymerisation von

a) mindestens einem Vinylester von $C_1$-$C_{24}$-Carbonsäuren in Gegenwart von
b) polyetherhaltigen Verbindungen mit einem Molekulargewicht von mindestens 300,

und mindestens eines weiteren copolymerisierbaren Monomeren c), ausgewählt aus der Gruppe der Monomere Methylmethacrylat, N-Vinylpyrrolidon, 3-Methyl-1-vinyl-imidazolium-methylsulfat N-Vinylformamid, Pentaerythrit-triallylether und N, N'-Divinyl-ethylenharnstoff in hautkosmetischen Formulierungen.

Bei der Herstellung der erfindungsgemäß verwendeten Polymerisate kann es während der Polymerisation zu einer Pfropfung auf die polyetherhaltigen Verbindungen (b) kommen, was zu den vorteilhaften Eigenschaften der Polymerisate führen kann. Es sind jedoch auch andere Mechanismen als Pfropfung vorstellbar.

Je nach Pfropfungsgrad sind unter den erfindungsgemäß verwendeten Polymerisaten sowohl reine Pfropfpolymerisate als auch Mischungen der o.g. Pfropfpolymerisate mit ungepfropften polyetherhaltigen Verbindungen und Homo- oder Copolymerisaten der Monomeren a) und c) zu verstehen.

Als polyetherhaltige Verbindungen (b) können sowohl Polyalkylenoxide auf Basis von Ethylenoxid, Propylenoxid, Butylenoxid und weiteren Alkylenoxiden als auch Polyglycerin verwendet werden. Je nach Art der Monomerbausteine enthalten die Polymere folgende Struktureinheiten.

$$-(CH_2)_2\text{-O-}, -(CH_2)_3\text{-O-}, -(CH_2)_4\text{-O-}, -CH_2\text{-}CH(R^6)\text{-O-},$$

$$-\ CH_2\text{-}CHOR^7\text{-}CH_2\text{-O-}$$

mit

$R^6$ $C_1$-$C_{24}$-Alkyl;

$R^7$ Wasserstoff, $C_1$-$C_{24}$-Alkyl, $R^6$-C(=O)-, $R^6$-NH-C(=O)-.

Dabei kann es sich bei den Struktureinheiten sowohl um Homopolymere als auch um statistische Copolymere und Blockcopolymere handeln.

Bevorzugt werden als Polyether (b) Polymerisate der allgemeinen Formel I verwendet,

$$R^1\left(-O-(R^2-O)_u-(R^3-O)_v-(R^4-O)_w\left[-A-R^2-O)_x-(R^3-O)_y-R^4-O)_z\right]_s-R^5\right)_n$$

(I)

in der die Variablen unabhängig voneinander folgende Bedeutung haben:

| | |
|---|---|
| $R^1$ | Wasserstoff, $C_1$-$C_{24}$-Alkyl, $R^6$-C(=O)-, $R^6$-NH-C(=O)-, Polyalkoholrest; |
| $R^5$ | Wasserstoff, $C_1$-$C_{24}$-Alkyl, $R^6$-C(=O)-, $R^6$-NH-C(=O)-; |
| $R^2$ bis $R^4$ | $-(CH_2)_2-$, $-(CH_2)_3-$, $-(CH_2)_4-$, $-CH_2-CH(R^6)-$, $-CH_2-CHOR^7-CH_2-$; |
| $R^6$ | $C_1$-$C_{24}$-Alkyl; |
| $R^7$ | Wasserstoff, $C_1$-$C_{24}$-Alkyl, $R^6$-C(=O)-, $R^6$-NH-C(=O)-; |
| A | -C(=O)-O, -C(=O)-B-C(=O)-O,<br>- C(=O)-NH-B-NH-C(=O)-O; |
| B | $-(CH_2)_t-$, Arylen, ggf. substituiert; |
| n | 1 bis 1000; |
| s | 0 bis 1000; |
| t | 1 bis 12; |
| u | 1 bis 5000; |
| v | 0 bis 5000; |
| w | 0 bis 5000: |
| x | 0 bis 5000; |
| y | 0 bis 5000; |
| z | 0 bis 5000. |

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge $C_1$-$C_{24}$ bzw. mit Carbonsäuren einer Kettenlänge $C_1$-$C_{24}$ verethert bzw. verestert werden oder mit Isocyanaten zu Urethanen umgesetzt werden.

Als Alkylreste für $R^1$ und $R^5$ bis $R^7$ seien verzweigte oder unverzweigte $C_1$-$C_{24}$-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als bevorzugte Vertreter der oben genannten Alkylreste seien verzweigte oder unverzweigte $C_1$-$C_{12}$-, beson-

ders bevorzugt $C_1$-$C_6$-Alkylketten genannt.

Das Molekulargewicht der Polyether beträgt mindestens 300 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen b) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z. B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Besonders bevorzugt werden als Polyether (b) Polymerisate der allgemeinen Formel I mit einem mittleren Molekulargewicht von 300 bis 100000 (nach dem Zahlenmittel), in der die Variablen unabhängig voneinander folgende Bedeutung haben:

| | |
|---|---|
| $R^1$ | Wasserstoff, $C_1$-$C_{12}$-Alkyl, $R^6$-C(=O)-, $R^6$-NH-C(=O)-, Polyalkoholrest; |
| $R^5$ | Wasserstoff, $C_1$-$C_{12}$-Alkyl, $R^6$-C(=O)-, $R^6$-NH-C(=O)-; |
| $R^2$ bis $R^4$ | $-(CH_2)_2-$, $-(CH_2)_3-$, $-(CH_2)_4-$, $-CH_2-CH(R^6)-$, $-CH_2-CHOR^7-CH_2-$; |
| $R^6$ | $C_1$-$C_{12}$-Alkyl; |
| $R^7$ | Wasserstoff, $C_1$-$C_{12}$-Alkyl, $R^6$-C(=O)-, $R^6$-NH-C(=O)-; |
| n | 1 bis 8; |
| s | 0; |
| u | 2 bis 2000; |
| v | 0 bis 2000; |
| w | 0 bis 2000. |

Ganz besonders bevorzugt werden als Polyether b) Polymerisate der allgemeinen Formel I mit einem mittleren Molekulargewicht von 500 bis 50000 (nach dem Zahlenmittel), in der die Variablen unabhängig voneinander folgende Bedeutung haben:

| | |
|---|---|
| $R^1$ | Wasserstoff, $C_1$-$C_6$-Alkyl, $R^6$-C(=O)-, $R^6$-NH-C(=O)-; |
| $R^5$ | Wasserstoff, $C_1$-$C_6$-Alkyl, $R^6$-C(=O)-, $R^6$-NH-C(=O)-; |
| $R^2$ bis $R^4$ | $-(CH_2)_2-$, $-(CH_2)_3-$, $-(CH_2)_4-$, $-CH_2-CH(R^6)-$, $-CH_2-CHOR^7-CH_2-$; |
| $R^6$ | $C_1$-$C_6$-Alkyl; |

$R^7$ Wasserstoff, $C_1$-$C_6$-Alkyl, $R^6$-C(=O)-, $R^6$-NH-C(=O)-;

n 1;

s 0;

u 5 bis 500;

v 0 bis 500;

w 0 bis 500.

Als Polyether können auch Silikonderivate eingesetzt werden. Geeignete Silikonderivate sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhône-Poulenc), Silicone Polyol Copolymer® (der Fa Genesee), Belsil® (der Fa. Wacker), Silwet® (der Fa. Witco, Greenwich, CT, USA) oder Dow Corning (der Fa. Dow Corning) erhältlich. Diese beinhalten Verbindungen mit den CAS-Nummern 64365-23-7; 68937-54-2; 68938-54-5; 68937-55-3.

Silikone werden in der Hautkosmetik im allgemeinen zur Verbesserung des Hautgefühls eingesetzt. Die Verwendung von polyetherhaltigen Silikonderivaten als Polyether (b) in den erfindungsgemäßen Polymeren kann deshalb zusätzlich zu einer Verbesserung des Hautgefühls führen.

Bevorzugte Vertreter solcher polyetherhaltigen Silikonderivaten sind solche, die die folgenden Strukturelemente enthalten:

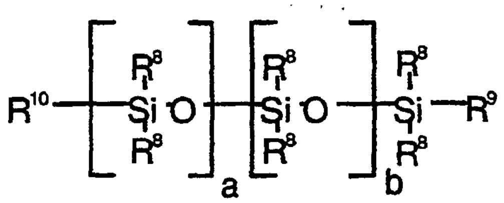

(II)

wobei:

$R^9$ = $CH_3$ oder 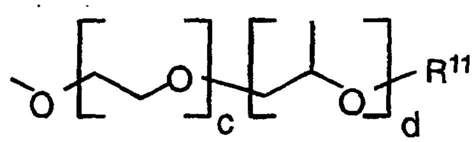

$R^{10}$ = $CH_3$ oder $R^9$

$R^{11}$ = H, $CH_3$, 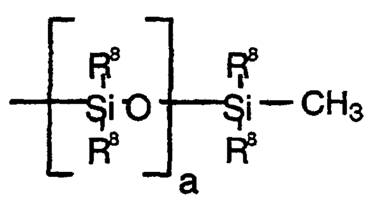

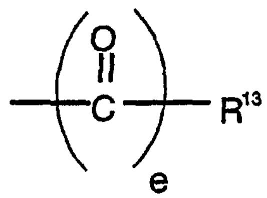

$R^{13}$ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,

und wobei die Reste $R^8$ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich $R^{12}$ sind, wobei:

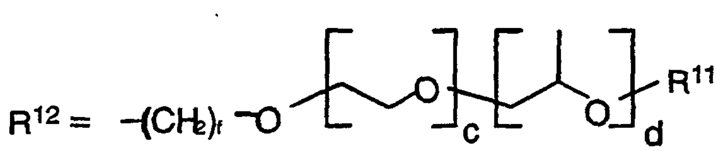

mit der Maßgabe, daß mindestens einer der Reste $R^8$, $R^9$ oder $R^{10}$ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist,
und e 0 oder 1 ist.

Bevorzugte Reste $R^9$ und $R^{12}$ sind solche, bei denen die Summe aus c+d zwischen 5 und 30 beträgt.

Bevorzugt werden die Gruppen $R^8$ aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und $R^{12}$.

Besonders geeignete Reste $R^{11}$ sind solche, bei denen im Falle von $R^{11} = -(CO)_e-R^{13}$ $R^{13}$ ein beliebiger Alkyl-, Cycloalkyl oder Arylrest bedeutet, der zwischen 1 und 40 C-Atomen besitzt und der weitere ionogene Gruppen wie $NH_2$, COOH, $SO_3H$ tragen kann.

Bevorzugte anorganische Reste $R^{13}$ sind, für den Fall e=0, Phosphat und Sulfat.

Besonders bevorzugte polyetherhaltige Silikonderivate sind solche der allgemeinen Struktur:

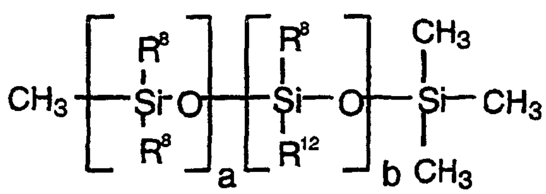

Des weiteren können als Polyether (b) auch Homo- und Copolymerisate aus polyalkylenoxidhaltigen ethylenisch ungesättigten Monomeren wie beispielsweise Polyalkylenoxid(meth)acrylate, Polyalkylenoxidvinylether, Polyalkylenoxid(meth)acrylamide, Polyalkylenoxidallylamide oder Polyalkylenoxidvinylamide verwendet werden. Selbstverständlich können auch Copolymerisate solcher Monomere mit anderen ethylenisch ungesättigten Monomeren eingesetzt werden.

Als polyetherhaltige Verbindungen b) können aber auch Umsetzungsprodukte von Polyethyleniminen mit Alkylenoxiden eingesetzt werden. Als Alkylenoxide werden in diesem Fall bevorzugt Ethylenoxid, Propylenoxid, Butylenoxid und Mischungen aus diesen, besonders bevorzugt Ethylenoxid verwendet. Als Polyethylenimine können Polymere mit zahlenmittleren Molekulargewichten von 300 bis 20000, bevorzugt 500 bis 10000, ganz besonders bevorzugt 500 bis 5000, eingesetzt werden. Das Gewichtsverhältnis zwischen eingesetztem Alkylenoxid und Polyethylenimin liegt im Bereich von 100 : 1 bis 0,1 : 1, bevorzugt im Bereich 50 : 1 bis 0,5 : 1, ganz besonders bevorzugt im Bereich 20 : 1 bis 0,5 : 1.

Für die Polymerisation in Gegenwart der Polyether b) seien als Komponente a) folgende radikalisch polymerisierbare Monomere genannt: Vinylester von aliphatischen, gesättigten oder ungesättigten $C_1$-$C_{24}$-Carbonsäuren, wie z.B. Ameisensäure, Essigsäure, Propionsäure, Buttersäure, Valeriansäure, Isovaleriansäure, Capronsäure, Caprylsäure, Caprinsäure, Undecylensäure, Laurinsäure, Myristinsäure, Palmitinsäure, Palmitoleinsäure, Stearinsäure, Ölsäure, Arachinsäure, Behensäure, Lignocerinsäure, Cerotinsäure sowie Melissensäure.

Bevorzugt werden Vinylester der oben genannten $C_1$-$C_{12}$-Carbonsäuren, insbesondere der $C_1$-$C_6$-Carbonsäuren, verwendet. Ganz besonders bevorzugt ist Vinylacetat.

Selbstverständlich können auch Mischungen der jeweiligen Monomeren aus der Gruppe a) copolymerisiert werden.

Die Vinylester (a) können daneben auch in Mischung mit einem oder mehreren, ethylenisch ungesättigten copolymerisierbaren Comonomeren (c) eingesetzt werden, wobei der Anteil dieser zusätzlichen Monomeren auf maximal 50 Gew.-% beschränkt sein sollte. Bevorzugt sind Anteile von 0 bis 20 Gew.-%. Der Begriff ethylenisch ungesättigt bedeutet, daß die Monomere zumindest eine radikalisch polymerisierbare Kohlenstoff-Kohlenstoff Doppelbindung besitzen, die mono-, di-, tri- oder tetrasubstituiert sein kann.

Die bevorzugten zusätzlich eingesetzten ethylenisch ungesättigten Comonomere (c) können durch die folgende allgemeine Formel beschrieben werden:

$$X\text{-}C\ (O)\ CR^{15}{=}CHR^{14}$$

wobei

X ausgewählt ist aus der Gruppe der Reste -OH, -OM, $-OR^{16}$, $NH_2$, $-NHR^{16}$, $N(R^{16})_2$ ;

M ist ein Kation ausgewählt aus der Gruppe bestehend aus: $Na^+$, $K^+$, $Mg^{++}$, $Ca^{++}$, $Zn^{++}$, $NH_4^+$, Alkylammonium, Dialkylammonium, Trialkylammonium und Tetraalkylammonium;

die Reste $R^{16}$ können identisch oder verschieden ausgewählt werden aus der Gruppe bestehend aus -H, $C_1$-$C_{40}$ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl.

$R^{15}$ und $R^{14}$ sind unabhängig voneinander ausgewählt aus der Gruppe bestehend aus: -H, $C_1$-$C_8$ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (c) sind zum Beispiel Acrylsäure oder Methacrylsäure und deren Salze, Ester und Amide. Die Salze können von jedem beliebigen nicht toxischen Metall, Ammonium oder substituierten Ammonium-Gegenionen abgeleitet sein.

Die Ester können abgeleitet sein von $C_1$-$C_{40}$ linearen, $C_3$-$C_{40}$ verzweigtkettigen oder $C_3$-$C_{40}$ carbocyclischen Alkoholen, von mehrfachfunktionellen Alkoholen mit 2 bis etwa 8 Hydroxylgruppen wie Ethylenglycol, Hexylenglycol, Glycerin und 1,2,6-Hexantriol, von Aminoalkoholen oder von Alkoholethern wie Methoxyethanol und Ethoxyethanol, (Alkyl)Polyethylenglykolen, (Alkly)Polypropylenglykolen oder ethoxylierten Fettalkoholen, beispielsweise $C_{12}$-$C_{24}$-Fettalkoholen umgesetzt mit 1 bis 200 Ethylenoxid-Einheiten.

Ferner eignen sich N,N-Dialkylaminoalkylacrylate- und -methacrylate und N-Dialkylaminoalkylacryl- und -methacrylamide der allgemeinen Formel (III)

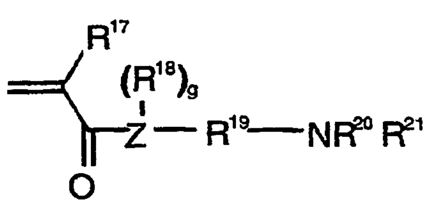

(III)

mit

| | |
|---|---|
| $R^{17}$ = | H, Alkyl mit 1 bis 8 C-Atomen, |
| $R^{18}$ = | H, Methyl, |
| $R^{19}$ = | Alkylen mit 1 bis 24 C-Atomen, optional substituiert durch Alkyl, |
| $R^{20}$, $R^{21}$ = | $C_1$-$C_{40}$ Alkylrest, |

Z = Stickstoff für g = 1 oder Sauerstoff für g = 0

Die Amide können unsubstituiert, N-Alkyl oder N-Alkylamino monosubstituiert oder N,N-dialkylsubstituiert oder N,N-dialkylaminodisubstituiert vorliegen, worin die Alkyl- oder Alkylaminogruppen von $C_1$-$C_{40}$ linearen, $C_3$-$C_{40}$ verzweigtkettigen, oder $C_3$-$C_{40}$ carbocyclischen Einheiten abgeleitet sind. Zusätzlich können die Alkylaminogruppen quaternisiert werden.

Bevorzugte Comonomere der Formel III sind N,N-Dimethylaminomethyl(meth)acrylat, N,N-Diethylaminomethyl(meth)acrylat, N,N-Dimethylaminoethyl(meth)acrylat, N,N-Diethylaminoethyl-(meth)acrylat, N-[3-(dimethylamino)propyl]methacrylamid und N-[3-(dimethylamino)propyl]acrylamid.

Ebenfalls verwendbare Comonomere (c) sind substituierte Acrylsäuren sowie Salze, Ester und Amide davon, wobei die Substituenten an den Kohlenstoffatomen in der zwei oder drei Position der Acrylsäure stehen, und unabhängig voneinander ausgewählt sind aus der Gruppe bestehend aus $C_1$-$C_4$ Alkyl, -CN, COOH besonders bevorzugt Methacrylsäure, Ethacrylsäure und 3-Cyanoacrylsäure. Diese Salze, Ester und Amide dieser substituierten Acrylsäuren können wie oben für die Salze, Ester und Amide der Acrylsäure beschrieben ausgewählt werden.

Andere geeignete Comonomere (c) sind Allylester von $C_1$-$C_{40}$ linearen, $C_3$-$C_{40}$ verzweigtkettigen oder $C_3$-$C_{40}$ carbocyclische Carbonsäuren, Vinyl- oder Allylhalogenide, bevorzugt Vinylchlorid und Allylchlorid, Vinylether, bevorzugt Methyl-, Ethyl-, Butyl- oder Dodecylvinylether, Vinylformamid, Vinylmethylacetamid, Vinylamin; Vinyllactame, bevorzugt Vinylpyrrolidon und Vinylcaprolactam, Vinyl- oder Allyl-substituierte heterocyclische Verbindungen, bevorzugt Vinylpyridin, Vinyloxazolin und Allylpyridin.

Weiterhin sind N-Vinylimidazole der allgemeinen Formel IV geeignet, worin $R^{22}$ bis $R^{24}$ unabhängig voneinander für Wasserstoff, $C_1$-$C_4$-Alkyl oder Phenyl steht:

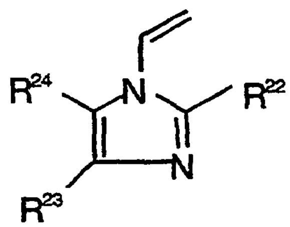

(IV)

Weitere geeignete Comonomere (c) sind Diallylamine der allgemeinen Formel (V)

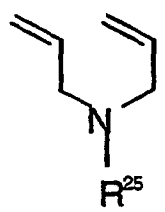

(V)

mit $R^{25}$ = $C_1$- bis $C_{24}$-Alkyl

Weitere geeignete Comonomere (c) sind Vinylidenchlorid; und Kohlenwasserstoffe mit mindestens einer Kohlenstoff-Kohlenstoff Doppelbindung, bevorzugt Styrol, alpha-Methylstyrol, tert.-Butylstyrol, Butadien, Isopren, Cyclohexadien, Ethylen, Propylen, 1-Buten, 2-Buten, Isobutylen, Vinyltoluol, sowie Mischungen dieser Monomere.

Besonders geeignete Comonomere (c) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butylethacrylat, 2-Ethylhexylethacrylat, Decylethacrylat, Stearyl(meth)acrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren wie zum Beispiel Acrylamidopropansulfonsäure;

Acrylamid, Methacrylamid, Ethacrylamid, N-Methylacrylamid, N,N-Dimethylacrylamid, N-Ethylacrylamid,

N-Isopropylacrylamid, N-Butylacrylamid, N-t-Butylacrylamid, N-Octylacrylamid, N-t-Octylacrylamid, N-Octadecylacrylamid, N-Phenylacrylamid, N-Methylmethacrylamid, N-Ethylmethacrylamid, N-Dodecylmethacrylamid, 1-Vinylimidazol, 1-Vinyl-2-methylvinylimidazol, N,N-Dimethylaminomethyl(meth)acrylat, N,N-Diethylaminomethyl(meth)-acrylat, N, N-Dimethylaminoethyl(meth)acrylat, N,N-Diethylaminoethyl(meth)acrylat, N,N-Dimethylaminobutyl(meth)acrylat, N, N-Diethylaminobutyl(meth)acrylat, N,N-Dimethylaminohexyl(meth)-acrylat, N,N-Dimethylaminooctyl(meth)acrylat, N, N-Dimethylaminododecyl(meth)acrylat, N-[3-(dimethylamino)propyl]methacrylamid, N-[3-(dimethylamino)propyl] acrylamid, N-[3-(dimethylamino)-butyl]methacrylamid, N-[8-(dimethylamino)octyl]methacrylamid, N-[12-(dimethylamino)dodecyl]methacrylamid, N-[3-(diethylamino)-propyl]methacrylamid, N-[3-(diethylamino)propyl]acrylamid;

Maleinsäure, Fumarsäure, Maleinsäureanhydrid und seine Halbester, Crotonsäure, Itaconsäure, Diallyldimethylammoniumchlorid, Vinylether (zum Beispiel: Methyl-, Ethyl-, Butyl- oder Dodecylvinylether), Vinylformamid, Vinylmethylacetamid, Vinylamin; Methylvinylketon, Maleimid, Vinylpyridin, Vinylimidazol, Vinylfuran, Styrol, Styrolsulfonat, Allylalkohol, und Mischungen daraus.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, Isobutylacrylat, Isobutylmethacrylat, 2-Ethylhexylacrylat, Stearylacrylat, Stearylmethacrylat, N-t-Butylacrylamid, N-Octylacrylamid, 2-Hydroxyethylacrylat, Hydroxypropylacrylate, 2-Hydroxyethylmethacrylat, Hydroxypropylmethacrylate, Alkylenglykol(meth)acrylate, Styrol, ungesättigte Sulfonsäuren wie zum Beispiel Acrylamidopropansulfonsäure, Vinylpyrrolidon, Vinylcaprolactam, Vinylether (z.B.: Methyl-, Ethyl-, Butyl- oder Dodecylvinylether), Vinylformamid, Vinylmethylacetamid, Vinylamin, 1-Vinylimidazol, 1-Vinyl-2-methylimidazol, N,N-Dimethylaminomethylmethacrylat und N-[3-(dimethylamino)-propyl]methacrylamid; 3-Methyl-1-vinylimidazoliumchlorid, 3-Methyl-3-vinylimidazoliummethylsulfat, N,N-Dimethylaminoethylmethacrylat, N-[3-(dimethylamino)propyl]methacrylamid quaternisiert mit Methylchlorid, Methylsulfat oder Diethylsulfat.

Monomere, mit einem basischen Stickstoffatom, können dabei auf folgende Weise quarternisiert werden:

Zur Quaternisierung der Amine eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternierung der basischen Amine kann auch mit Alkylenoxiden wie Ethylenoxid oder Propylenoxid in Gegenwart von Säuren durchgeführt werden. Bevorzugte Quaternierungsmittel sind: Methylchlorid, Dimethylsulfat oder Diethylsulfat.

Die Quaternisierung kann vor der Polymerisation oder nach der Polymerisation durchgeführt werden.

Außerdem können die Umsetzungsprodukte von ungesättigten Säuren, wie z.B. Acrylsäure oder Methacrylsäure, mit einem quaternisierten Epichlorhydrin der allgemeinen Formel (VI) eingesetzt werden ($R^{26}$ = $C_1$- bis $C_{40}$-Alkyl).

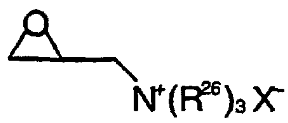

(VI)

Beispiele hierfür sind:

(Meth)acryloyloxyhydroxypropyltrimethylammoniumchlorid und
(Meth)acryloyloxyhydroxypropyltriethylammoniumchlorid.

Die basischen Monomere können auch kationisiert werden, indem sie mit Mineralsäuren, wie z.B. Schwefelsäure, Chlorwasserstoffsäure, Bromwasserstoffsäure, Iodwasserstoffsäure, Phosphorsäure oder Salpetersäure, oder mit organischen Säuren, wie z.B. Ameisensäure, Essigsäure, Milchsäure, oder Citronensäure, neutralisiert werden.

Zusätzlich zu den oben genannten Comonomeren können als Comonomere (c) sogenannte Makromonomere wie zum Beispiel silikonhaltige Makromonomere mit ein oder mehreren radikalisch polymerisierbaren Gruppen oder Alkyloxazolinmakromonomere eingesetzt werden, wie sie zum Beispiel in der EP 408 311 beschrieben sind.

Des weiteren können fluorhaltige Monomere, wie sie beispielsweise in der EP 558423 beschrieben sind, vernetzend wirkende oder das Molekulargewicht regelnde Verbindungen in Kombination oder alleine eingesetzt werden.

Als Regler können die üblichen dem Fachmann bekannten Verbindungen, wie zum Beispiel Schwefelverbindungen (z.B.: Mercaptoethanol, 2-Ethylhexylthioglykolat, Thioglykolsäure oder Dodecylmercaptan), sowie Tribromchlormethan oder andere Verbindungen, die regelnd auf das Molekulargewicht der erhaltenen Polymerisate wirken,

verwendet werden.

Es können gegebenenfalls auch thiolgruppenhaltige Silikonverbindungen eingesetzt werden. Bevorzugt werden silikonfreie Regler eingesetzt.

Als vernetzende Monomere können Verbindungen mit mindestens zwei ethylenisch ungesättigten Doppelbindungen eingesetzt werden, wie zum Beispiel Ester von ethylenisch ungesättigten Carbonsäuren, wie Acrylsäure oder Methacrylsäure und mehrwertigen Alkoholen, Ether von mindestens zweiwertigen Alkoholen, wie zum Beispiel Vinylether oder Allylether.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl-1,3-pentandiol, 1,2-Cyclohexandiol, 1,4-Cyclohexandiol, 1,4-Bis(hydroxymethyl)cyclohexan, Hydroxypivalinsäure-neopentylglycolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thio-pentan-1,5-diol, sowie Polyethylenglykole, Polypropylenglykole und Polytetrahydrofurane mit Molekulargewichten von jeweils 200 bis 10 000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C3- bis C6-Carbonsäuren, beispielsweise Acrylsäure, Methacrylsäure, Itaconsäure, Maleinsäure oder Fumarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Außerdem geeignet sind geradkettige oder verzweigte, lineare oder cyclische aliphatische oder aromatische Kohlenwasserstoffe, die über mindestens zwei Doppelbindungen verfügen, welche bei den aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Ferner geeignet sind Amide von ungesättigten Carbonsäuren, wie z.B., Acryl- und Methacrylsäure, Itaconsäure, Maleinsäure, und N-Allylaminen von mindestens zweiwertigen Aminen, wie zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin oder entsprechende Ammoniumsalze, z.B. Triallylmethylammoniumchlorid oder -methylsulfat, als Vernetzer geeignet.

Weiterhin können N-Vinylverbindungen von Harnstoffderivaten, mindestens zweiwertigen Amiden, Cyanuraten oder Urethanen, beispielsweise von Harnstoff, Ethylenharnstoff, Propylenharnstoff oder Weinsäurediamid, z.B. N, N'-Divinylethylenharnstoff oder N,N'-Divinylpropylenharnstoff eingesetzt werden.

Weitere geeignete Vernetzer sind Divinyldioxan, Tetraallylsilan oder Tetravinylsilan.

Besonders bevorzugte Vernetzer sind beispielsweise Methylenbisacrylamid, Divinylbenzol, Triallylamin und Triallylammoniumsalze, Divinylimidazol, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind, sowie Allyl- oder Vinylether von mehrwertigen Alkoholen, beispielsweise 1,2-Ethandiol, 1,4-Butandiol, Diethylenglykol, Trimethylolpropan, Glycerin, Pentaerythrit, Sorbitan und Zucker wie Saccharose, Glucose, Mannose.

Ganz besonders bevorzugt als Vernetzer sind Pentaerythrittriallylether, Allylether von Zuckern wie Saccharose, Glucose, Mannose, Divinylbenzol, Methylenbisacrylamid, N,N'-Divinylethylenharnstoff, und (Meth-)Acrylsäureester von Glykol, Butandiol, Trimethylolpropan oder Glycerin oder (Meth)Acrylsäureester von mit Ethylenoxid und/oder Epichlorhydrin umgesetzten Glykol, Butandiol, Trimethylolpropan oder Glycerin.

Der Anteil der vernetzend wirkenden Monomeren beträgt 0 bis 10 Gew.-%, bevorzugt 0 bis 5 Gew.-%, ganz besonders bevorzugt 0 bis 2 Gew.-%.

Bei der Polymerisation zur Herstellung der erfindungsgemäßen Polymerisate können gegebenenfalls auch andere Polymere, wie zum Beispiel Polyamide, Polyurethane, Polyester, Homo- und Copolymere von ethylenisch ungesättigten Monomeren, Polyvinylalkohol, zugegen sein. Beispiele für solche zum Teil auch in der Kosmetik eingesetzten Polymeren sind die unter den Handelsnamen bekannten Polymere Amerhold™, Ultrahold™, Luviquat™, Luviquat Care™, Ultrahold Strong™, Luviflex™ VBM, Luvimer™, Acronal™, Acudyne™, Stepanhold™, Lovocryl™, Versatyl™, Amphomer™ oder Eastma AQ™.

Die erfindungsgemäßen Comonomere (c) können, sofern sie ionisierbare Gruppen enthalten, vor oder nach der Polymerisation, zum Teil oder vollständig mit Säuren oder Basen neutralisiert werden, um so zum Beispiel die Wasserlöslichkeit oder -dispergierbarkeit auf ein gewünschtes Maß einzustellen.

Als Neutralisationsmittel für Säuregruppen tragende Monomere können zum Beispiel Mineralbasen wie Natriumcarbonat, Alkalihydroxide sowie Ammoniak, organische Basen wie Aminoalkohole speziell 2-Amino-2-Methyl-1-Propanol, Monoethanolamin, Diethanolamin, Triethanolamin, Triisopropanolamin, Tri[(2-hydroxy)1-Propyl]amin, 2-Amino-2-Methyl-1,3-Propandiol, 2-Amino-2-hydroxymethyl-1,3-Propandiol sowie Diamine, wie zum Beispiel Lysin, verwendet werden.

Zur Herstellung der Polymerisate können die Monomeren der Komponente a) in Gegenwart der Polyether sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azobis-(2-amidinopropan)dihydrochlorid oder 2-2'-Azo-bis-(2-methylbutyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumbydroxymethansulfinat.

Bevorzugt werden organische Peroxide eingesetzt.

Die verwendeten Mengen an Initiator bzw. Initiatorgemischen bezogen auf eingesetztes Monomer liegen zwischen 0,01 und 10 Gew.-%, vorzugsweise zwischen 0,1 und 5 Gew.-%.

Die Polymerisation erfolgt im Temperaturbereich von 40 bis 200°C, bevorzugt im Bereich von 50 bis 140°C, besonders bevorzugt im Bereich von 60 bis 110°C. Sie wird üblicherweise unter atmosphärischem Druck durchgeführt, kann jedoch auch unter vermindertem oder erhöhtem Druck, vorzugsweise zwischen 1 und 5 bar, ablaufen.

Die Polymerisation kann beispielsweise als Lösungspolymerisation, Polymerisation in Substanz, Emulsionspolymerisation, umgekehrte Emulsionspolymerisation, Suspensionspolymerisation, umgekehrte Suspensionspolymerisation oder Fällungspolymerisation durchgeführt werden, ohne daß die verwendbaren Methoden darauf beschränkt sind.

Bei der Polymerisation in Substanz kann man so vorgehen, daß man die polyetherhaltige Verbindung b) in mindestens einem Monomer der Gruppe a) und eventuell weiteren Comonomeren der Gruppe c) löst und nach Zugabe eines Polymerisationsinitiators die Mischung auspolymerisiert. Die Polymerisation kann auch halbkontinuierlich durchgeführt werden, indem man zunächst einen Teil, z.B. 10 % des zu polymerisierenden Gemisches aus der polyetherhaltigen Verbindung b), mindestens einem Monomeren der Gruppe a), eventuell weiteren Comonomeren der Gruppe c) und Initiator vorlegt, das Gemisch auf Polymerisationstemperatur erhitzt und nach dem Anspringen der Polymerisation den Rest der zu polymerisierenden Mischung nach Fortschritt der Polymerisation zugibt. Die Polymerisate können auch dadurch erhalten werden, daß man die polyetherhaltigen Verbindungen der Gruppe b) in einem Reaktor vorlegt, auf die Polymerisationstemperatur erwärmt und mindestens ein Monomer der Gruppe a), eventuell weiteren Comonomeren der Gruppe c) und Polymerisationsinitiator entweder auf einmal, absatzweise oder vorzugsweise kontinuierlich zufügt und polymerisiert.

Falls gewünscht, kann die oben beschriebene Polymerisation auch in einem Lösemittel durchgeführt werden. Geeignete Lösemittel sind beispielsweise Alkohole, wie Methanol, Ethanol, n-Propanol, Isopropanol, n-Butanol, sek. -Butanol, tert.-Butanol, n-Hexanol und Cyclohexanol sowie Glykole, wie Ethylenglykol, Propylenglykol und Butylenglykol sowie die Methyl- oder Ethylether der zweiwertigen Alkohole, Diethylenglykol, Triethylenglykol, Glycerin und Dioxan. Die Polymerisation kann auch in Wasser als Lösemittel durchgeführt werden. In diesem Fall liegt zunächst eine Lösung vor, die in Abhängigkeit von der Menge der zugegebenen Monomeren der Komponente a) in Wasser mehr oder weniger gut löslich ist. Um wasserunlösliche Produkte, die während der Polymerisation entstehen können, in Lösung zu überführen, kann man beispielsweise organische Lösemittel zusetzen, wie einwertige Alkohole mit 1 bis 3 Kohlenstoffatomen, Aceton oder Dimethylformamid. Man kann jedoch auch bei der Polymerisation in Wasser so verfahren, daß man die wasserunlöslichen Polymerisate durch Zugabe üblicher Emulgatoren oder Schutzkolloide, z.B. Polyvinylalkohol, in eine feinteilige Dispersion überführt.

Als Emulgatoren verwendet man beispielsweise ionische oder nichtionische Tenside, deren HLB-Wert im

Bereich von 3 bis 13 liegt. Zur Definition des HLB-Werts wird auf die Veröffentlichung von W.C. Griffin, J. Soc. Cosmetic Chem., Band 5, 249 (1954), hingewiesen.

Die Menge an Tensiden, bezogen auf das Polymerisat, beträgt 0,1 bis 10 Gew.-%. Bei Verwendung von Wasser als Lösemittel erhält man Lösungen bzw. Dispersionen der Polymerisate. Sofern man Lösungen des Polymerisates in einem organischen Lösemittel herstellt bzw. in Mischungen aus einem organischen Lösemittel und Wasser, so verwendet man pro 100 Gew.-Teile des Polymerisates 5 bis 2000, vorzugsweise 10 bis 500 Gew.-Teile des organischen Lösemittels oder des Lösemittelgemisches.

Bevorzugt sind Polymere, die erhältlich sind durch radikalische Polymerisation von

| | | |
|---|---|---|
| a) | 10 - 98 Gew.-% | mindestens eines Vinylesters von $C_1$-$C_{24}$-Carbonsäuren in Gegenwart von |
| b) | 2 - 90 Gew.-% | mindestens einer polyetherhaltigen Verbindung und |
| c) | 0 - 50 Gew.-% | eines oder mehreren weiteren copolymerisierbaren Monomeren |

Besonders bevorzugt sind Polymere, die erhältlich sind durch radikalische Polymerisation von

| | | |
|---|---|---|
| a) | 50 - 97 Gew.-% | mindestens eines Vinylesters von $C_1$-$C_{24}$-Carbonsäuren in Gegenwart von |
| b) | 3 - 50 Gew.-% | mindestens einer polyetherhaltigen Verbindung und |
| c) | 0 - 30 Gew.-% | eines oder mehreren weiteren copolymerisierbaren Monomeren |

Ganz besonders bevorzugt sind Polymere, die erhältlich sind durch radikalische Polymerisation von

| | | |
|---|---|---|
| a) | 65 - 97 Gew.-% | mindestens eines Vinylesters von $C_1$-$C_{24}$-Carbonsäuren in Gegenwart von |
| b) | 3 - 40 Gew.-% | mindestens einer polyetherhaltigen Verbindung und |
| c) | 0 - 20 Gew.-% | eines oder mehreren weiteren copolymerisierbaren Monomeren |

Zur Herstellung der erfindungsgemäß verwendeten Polymeren können die Estergruppen der ursprünglichen Monomere a) und gegebenenfalls weiterer Monomere nach der Polymerisation durch Hydrolyse, Alkoholyse oder Aminolyse gespalten werden. Im nachfolgenden wird dieser Verfahrensschritt allgemein als Verseifung bezeichnet. Die Verseifung erfolgt in an sich bekannter Weise durch Zugabe einer Base, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base, von der Verseifungstemperatur, der Verseifungszeit und dem Wassergehalt der Lösung.

Der Verseifungsgrad der Polyvinylestergruppen kann bis zu 100 % betragen, bevorzugt ist ein Bereich von 40 bis 100 %, besonders bevorzugt ein Bereich von 65 bis 100 %, ganz besonders bevorzugt ein Bereich von 80 bis 100 %.

Die so hergestellten Polymerisate können durch Umsetzung von im Polymer vorhandenen Hydroxyl- und/oder Aminofunktionen mit Epoxiden der Formel VI nachträglich kationisiert werden ($R^{26}$ = $C_1$ bis $C_{40}$ Alkyl) .

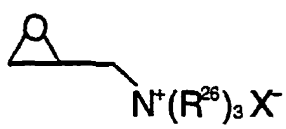

(VI)

Dabei können bevorzugt die Hydroxylgruppen der Polyvinylalkohol-Einheiten und Vinylamin-Einheiten, entstanden durch Hydrolyse von Vinylformamid, mit den Epoxiden umgesetzt werden.

Die Epoxide der Formel VI können auch in situ durch Umsetzung der entsprechenden Chlorhydrine mit Basen, beispielsweise Natriumhydroxid, erzeugt werden.

Bevorzugt wird 2,3-Epoxypropyl-trimethylammoniumchlorid bzw. 3-Chlor-2-hydroxypropyl-trimethylammoniumchlorid eingesetzt.

Die K-Werte der Polymerisate sollen im Bereich von 10 bis 300, bevorzugt 25 bis 250, besonders bevorzugt 25 bis 200, ganz besonders bevorzugt im Bereich von 30 und 150, liegen. Der jeweils gewünschte K-Wert läßt sich in an sich bekannter Weise durch die Zusammensetzung der Einsatzstoffe einstellen. Die K-Werte werden bestimmt nach Fikentscher, Cellulosechemie, Bd. 13, S. 58 bis 64, und 71 bis 74 (1932) in N-Methylpyrrolidon bei 25°C und Polymerkonzentrationen, die je nach K-Wert-Bereich zwischen 0,1 Gew.-% und 5 Gew.-% liegen.

Die Polymerlösungen können zur Entfernung von Lösungsmitteln wasserdampfdestilliert werden. Nach der Wasserdampfdestillation erhält man je nach Verseifungsgrad, Art der Polyether b), der Vinylester a) und der eventuell eingesetzten Monomere c) wäßrige Lösungen oder Dispersionen.

Die erhaltenen Polymerisate können auch nachträglich vernetzt werden, indem man die Hydroxylgruppen bzw. Aminogruppen im Polymer mit mindestens bifunktionellen Reagentien umsetzt. Bei niedrigen Vernetzungsgraden erhält man wasserlösliche Produkte, bei hohen Vernetzungsgrade wasserquellbare bzw. unlösliche Produkte.

Beispielsweise können die erfindungsgemäßen Polymerisate mit Dialdehyden und Diketonen, z.B. Glyoxal, Glutaraldehyd, Succindialdehyd oder Terephthalaldehyd, umgesetzt werden. Desweiteren eignen sich aliphatische oder aromatische Carbonsäuren, beispielsweise Maleinsäure, Oxalsäure, Malonsäure, Succinsäure oder Citronensäure, bzw. Carbonsäurederivaten wie Carbonsäureester, -anhydride oder -halogenide. Ferner sind mehrfunktionelle Epoxide geeignet, z.B. Epichlorhydrin, Glycidylmethacrylat, Ethylenglykoldiglycidylether, 1,4-Butandioldiglycidylether oder 1,4-Bis-(glycidyloxy)benzol. Ferner eigenen sich Diisocyanate, beispielsweise Hexamethylendiisocyanat, Isophorondiisocyanat, Methylendiphenyldiisocyanat, Toluylendiisocyanat oder Divinylsulfon. Weiterhin eignen sich anorganische Verbindungen wie Borsäure oder Borsäuresalze, beispielsweise Natriummetaborat, Borax (Dinatriumtetraborat), sowie Salze mehrwertiger Kationen, z B. Kupfer(II)salze wie Kupfer(II)acetat oder Zink-, Aluminium-, Titansalze. Borsäure bzw. Borsäuresalze wie Natriummetaborat oder Dinatriumtetraborat eignen sich bevorzugt zur nachträglichen Vernetzung. Dabei können die Borsäure bzw. Borsäuresalze, bevorzugt als Salzlösungen, den Lösungen der erfindungsgemäßen Polymerisate zugegeben werden. Bevorzugt werden die Borsäure bzw. Borsäuresalze den wässerigen Polymerisatlösungen hinzugefügt.

Die Borsäure bzw. Borsäuresalze können den Polymerlösungen direkt nach der Herstellung zugefügt werden. Es ist aber auch möglich, die Borsäure bzw. Borsäuresalze nachträglich den kosmetischen Formulierungen mit den erfindungsgemäßen Polymerisaten zuzusetzen, bzw. während des Herstellungsprozesses der kosmetischen Formulierungen.

Der Anteil Borsäure bzw. Borsäuresalze bezogen auf die erfindungsgemäßen Polymere beträgt 0 bis 15 Gew-%, bevorzugt 0 bis 10 Gew.-%, besonders bevorzugt 0 bis 5 Gew.-%.

Die Polymerisatlösungen und -dispersionen können durch verschiedene Trocknungsverfahren wie z.B. Sprühtrocknung, Fluidized Spray Drying, Walzentrocknung oder Gefriertrocknung in Pulverform überführt werden. Als Trocknungsverfahren wird bevorzugt die Sprühtrocknung eingesetzt. Aus dem so erhaltenen Polymer-Trockenpulver läßt sich durch Lösen bzw. Redispergieren in Wasser erneut eine wäßrige Lösung bzw. Dispersion herstellen. Die Überführung in Pulverform hat den Vorteil einer besseren Lagerfähigkeit, einer einfacheren Transportmöglichkeit sowie eine geringere Neigung für Keimbefall.

Anstelle der wasserdampfdestillierten Polymerlösungen können auch die alkoholischen Polymerlösungen direkt in Pulverform überführt werden.

Die erfindungsgemäßen Polymerisate eignen sich hervorragend zur Verwendung in hautkosmetischen Formulierungen, insbesondere zur Herstellung von Hautpflegemitteln wie Gesichts-, Augen- und Fußpflegemitteln, Körperlotionen, Duschgelen, Sonnenschutzmitteln und Mitteln zur dekorativen Hautkosmetik, sowie zur Herstellung von Hautpflegemitteln wie Tag- und Nachtcremes, Vitamincremes, Bleichcremes, WO- oder O/W-Hautcremes, Peelingscremes, Lippenstiften, Make-up, Gesichtsmasken, Peel-Off Masken und Maskara.

Weiterhin sind die erfindungsgemäßen Polymerisate geeignet zur Verwendung in Zahn- und Mundpflegemitteln wie Mundwasser und Zahnpasta sowie als Prothesenhaftmittel.

Die erfindungsgemäßen Copolymere sind in den Hautpflegemitteln in einem Anteil von etwa 0,001 bis 25 Gew.-%, vorzugsweise 0,05 bis 20 Gew.-%, bezogen auf das Gesamtgewicht des Mittels, enthalten.

Je nach Anwendungsgebiet können die erfindungsgemäßen Mittel in einer zur Hautpflege geeigneten Form, wie z.B. als Creme, Schaum, Gel, Stift, Pulver, Spray oder Mousse appliziert werden.

Die erfindungsgemäßen hautkosmetischen Formulierungen enthalten in einer bevorzugten Ausführungsform

| | | |
|---|---|---|
| a) | 0,05 - 20 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 20 - 99,95 Gew.-% | Wasser und/oder Lösungsmittel und/oder einer Ölkomponente |
| c) | 0 - 79,5 Gew.-% | weitere Bestandteile |

Als geeignete Lösungsmittel sind insbesondere zu nennen niedrige Monoalkohole oder Polyole mit 1 bis 6 Kohlenstoffatomen oder Mischungen davon; bevorzugte Monoalkohole oder Polyole sind Ethanol, i-Propanol, n-Propanol', Propylenglycol, Glycerin und Sorbit.

Als geeignete Fettkörper können enthalten sein mineralische, tierische, pflanzliche oder synthetische Öle

oder Wachse, Fettsäuren, Fettsäureester, wie Triglyceride von C6-C18-Fettsäuren, Fettalkohole, Vaseline, Paraffin, Lanolin, hydriertes Lanolin, azetyliertes Lanolin und Siliconöl.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Lichtschutzmittel, UV-Filter, kosmetische Wirkstoffe wie Phytantriol, Vitamin A, E und C, Retinol, Bisabolol, Panthenol, Collagen, Alpha- und Beta-Hydroxycarbonsäuren, Eiweißhydrolysate, Bleichmittel, Bräunungsmittel (z.B. Dihydroxyaceton), Stabilisatoren, pH-Wert-Regulatoren, Farbstoffe, Viskositätsregulierer, Gelbildner, Salze, Feuchthaltemittel, Rückfetter und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Kosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset® P.U.R.), Polyharnstoffe und Polyvinylalkohole. Besonders geeignete Polymere sind Copolymere aus t-Butylacrylat, Ethylacrylat, Methacrylsäure (z.B. Luvimer® 100P), Copolymere aus N-tert.-Butylacrylamid, Ethylacrylat, Acrylsäure (Ultrahold® 8, strong), Copolymere aus Vinylacetat, Crotonsäure und gegebenenfall weiteren Vinylestern (z.B. Luviset® Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol® VBM).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Hautkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Als weitere Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Copolymerisate werden in kosmetischen oder dermatologischen Zubereitungen eingesetzt, deren Herstellung nach den üblichen dem Fachmann geläufigen Regeln erfolgt.

Solche Formulierungen liegen vorteilhafterweise in Form von Emulsionen bevorzugt als Wasser-in-Öl-(W/O)- oder Öl-in-Wasser-(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden. Die Auswahl der Emulsionstypspezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

In einer bevorzugten Ausführungsform enthalten erfindungsgemäße Emulsionen

| | | |
|---|---|---|
| a) | 0,05 - 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 10 - 94,94 Gew.-% | Wasser |
| c) | 5 - 89,94 Gew.-% | einer Ölkomponente |
| d) | 0,01 - 40 Gew.-% | eines Emulgators |
| e) | 0 - 74,94 Gew.-% | weitere Bestandteile |

Eine bevorzugte Form einer hautkosmetischen Formulierung der erfindungsgemäßen Polymere ist eine W/O-Emulsion, die eine wässrige Phase enthält, die mittels eines geeigneten Emulgatorsystems in einer Öl- oder Fettphase emulgiert ist.

| | | |
|---|---|---|
| a) | 0,05 - 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 20 - 77,95 Gew.-% | Wasser |
| c) | 20 - 77,95 Gew.-% | einer Ölkomponente |
| d) | 2 - 35 Gew.-% | eines Emulgators |
| e) | 0 - 55,95 Gew.-% | weitere Bestandteile |

Bei den Emulgatoren handelt es sich um diejenigen, welche in diesem Emulsionstyp üblicherweise verwendet werden. Sie werden z.B. ausgewählt unter: $C_{12}$-$C_{18}$-Sorbitan-Fettsäureestern; Estern von Hydroxystearinsäure und $C_{12}$-$C_{30}$-Fettalkoholen; Mono- und Diestern von $C_{12}$-$C_{18}$-Fettsäuren und Glyzerin oder Polyglyzerin; Kondensaten von Ethylenoxid und Propylenglycolen; oxypropylenierten/oxyethylenierten $C_{12}$-$C_{20}$-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Zu geeigneten Fettkomponenten, welche in der Fettphase der Emulsionen enthalten sein können, zählen: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250oC und deren Destillationsendpunkt bei 410oC liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwadhs und Ca-, Mg- und Al-Oleate, -Myristate, -Linoleate und -Stearate.

Im Allgemeinen werden diese Wasser-in-Öl-Emulsionen so hergestellt, dass die Fettphase und der Emulgator in den Ansatzbehälter gegeben werden. Man erwärmt diesen bei einer Temperatur von 70 bis 75oC, gibt dann die in Öl löslichen Ingredienzien zu und fügt unter Rühren Wasser hinzu, welches vorher auf die gleiche Temperatur erwärmt wurde und worin man die wasserlöslichen Ingredienzien vorher gelöst hat; man rührt, bis man eine Emulsion der gewünschten Feinheit hat, läßt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die gegebenenfalls verdickt vorliegt.

| | | |
|---|---|---|
| a) | 0,05 - 10 Gew.-% | des erfindungsgemäßen Polymeren, hergestellt durch radikalische Polymerisation von Vinylestern und optional weiterer polymerisierbarer Monomerer in Gegenwart polyetherhaltiger Verbindungen |
| b) | 40 - 96,95 Gew.-% | Wasser |
| c) | 1 - 44,95 Gew.-% | einer Ölkomponente |
| d) | 1 - 35 Gew.-% | eines Emulgators |
| e) | 0 - 10 Gew.-% | eines Gelbildners |
| f) | 0 - 57,95 Gew.-% | weitere Bestandteile |

Als Emulgatoren kommen vorzugsweise O/W-Emulgatoren in Betracht. Geeignete Emulgatoren können nichtionisch, kationisch bzw. anionisch sein.

Beispiele für nichtionische Emulgatoren (INCI-Nomenklatur) sind Laurethe, z.B. Laureth-4; Cetethe, z.B. Ceteth-1, Polyethylenglycolcetylether; Cetearethe, z.B. Ceteareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat,

Cetyltrimoniumchlorid, Cetyltrimoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Zu geeigneten Ölkomponenten, welche in der Fettphase der Emulsionen enthalten sein können, zählen: Kohlenwasserstofföle, wie Paraffinöl, Purcellinöl, Perhydrosqualen und Lösungen mikrokristalliner Wachse in diesen Ölen; Ester aus gesättigten und/oder ungesättigten, verzweigten und/oder unverzweigten $C_3$-$C_{30}$-Alkancarbonsäuren und gesättigten und/oder ungesättigten, verzweigten und/oder unverzweigten $C_3$-$C_{30}$-Alkoholen, aus aromatischen Carbonsäuren und gesättigten und/oder ungesättigten, verzweigten und/oder unverzweigten $C_3$-$C_{30}$-Alkoholen, beispielhaft Isopropylstearat, Hexyldecylstearat, Oleyloleat, i-Propyl-, Butyl- oder Cetylmyristat, Ethyl- oder i-Propylpalmitat, Octan- oder Decansäuretriglyceride und Cetylricinoleat.; außerdem synthetische, halbsynthetische und natürliche Gemische solcher Ester, wie Jojobaöl, tierische oder pflanzliche Öle, wie Süßmandelöl, Avocadoöl, Calophylumöl, Lanolin und Derivate davon, Ricinusöl, Sesamöl, Palmöl, Mandelöl, Olivenöl, Jojobaöl, Karite-Öl, Hoplostethus-Öl; mineralische Öle, deren Destillationsbeginn unter Atmosphärendruck bei ca. 250oC und deren Destillationsendpunkt bei 410oC liegt, wie z.B. Vaselinöl.

Die Fettphase kann auch in anderen Ölen lösliche Siliconöle, wie Dimethylpolysiloxan, Cyclomethicon, Methylphenylpolysiloxan und das Siliconglycol-Copolymer, Octamethylcyclotetrasiloxan enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwadhs und Ca-, Mg- und Al-Oleate, -Myristate, -Linoleate und -Stearate.

Als übliche Verdickungsmittel bzw. Gelbildner können beispielsweise vernetzte Polyacrylsäuren und deren Derivate, Polysaccharide wie Xan-than Gum, oder Hydroxycarboxymethylcellulose, Fettalkohole eingesetzt werden.

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, Carboxymethylcellulose, kationisch modifizierte Cellulosen, Polysaccharide, z.B. Xanthum Gummi oder Alginate, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycol Dicaprate Dicaprylate (and) PPG-1 Trideceth-6, Polyquaternium-7, Polyquaternium-44, Polyvinylalkohol und Polyvinylpyrrolidon.

Die Herstellung kann durch Aufschmelzen der Ölphase bei ca. 80°C erfolgen; die wasserlöslichen Bestandteile werden in heißem Wasser gelöst, und langsam unter Rühren die aufgeschmolzene Ölphase zugegeben; homogenisiert und kaltgerührt.

Die erfindungsgemäßen Copolymerisate eignen sich auch zur Verwendung in Wasch- und Duschgel-Formulierungen sowie Badepräparaten.

Solche Formulierungen enthalten neben den erfindungsgemäßen Polymeren üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

Die Formulierungen enthalten 2 bis 50 Gew.-% Tenside, bevorzugt 5 bis 40 Gew.-%, besonders bevorzugt 8 bis 30 Gew-%.

In den Wasch- und Duschgel-Formulierungen sowie Badepräparaten können alle üblicherweise zur Hautreinigung eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglykoside oder Sorbitanetherester geeignet.

Außerdem können die Wasch- und Duschgel-Formulierungen sowie Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46) und andere.

Weiterhin können die Wasch- und Duschgel-Formulierungen sowie Badepräparaten Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

Herstellungsbeispiele:

Herstellvorschrift für Polymere:

In einem Polymerisationsgefäß wird die polyetherhaltige Verbindung vorgelegt und unter Rühren und leichtem Stickstoffstrom auf 80°C erhitzt. Unter Rühren werden Vinylacetat und gegebenenfalls die weiteren Monomeren in 3 h zudosiert. Gleichzeitig wird eine Lösung von 1,4 g tert.-Butylperpivalat in 30 g Methanol ebenfalls in 3 h zugegeben. Danach wird noch 2 h bei 80°C gerührt. Nach dem Abkühlen wird das Polymerisat in 450 ml Methanol gelöst. Zur Verseifung gibt man bei 30°C 50 ml einer 10%igen methanolischen Natriumhydroxidlösung zu. Nach ca. 40 min. wird die Reaktion durch Zugabe von 750 ml 1%iger Essigsäure abgebrochen. Das Methanol wird durch Destillation entfernt.

Die K-Werte wurden 1%ig in N-Methylpyrrolidon bestimmt.

Tabelle

| Beispiel | Pfropfgrundlage | Vinylester | Comonomer | K-Wert | Verseifungsgrad[%] |
|---|---|---|---|---|---|
| 23 | PEG 6000,[1] 72 g | Vinylacetat, 386 g | Methylmethacrylat, 24 g | 47 | >95 |
| 24 | PEG 20000, 72 g | Vinylacetat, 328 g | N-Vinylpyrrolidon, 82 | 61 | >95 |
| 25 | PEG 20000, 72 g | Vinylacetat, 362 g | 3-Methyl-1-vinyl-imidazoliummethylsulfat, 48 g | 53 | >95 |
| 26 | PEG 6000, 72 g | Vinylacetat, 367 g | N-Vinylformamid, 41 g | 57 57 | >95 >95 |
| 27 | PEG 6000, 72 g | Vinylacetat, 326 g | N-Vinylformamid, 82 g | 67 67 | >95 >95 |
| 29 | PEG 35000, 270 g | Vinylacetat, 410 g | Pentaerythrittriallyl-ether, 1,6 g | 71 | 95 |
| 30 | PEG 35000, 270 g | Vinylacetat, 410 g | Pentaerythrittriallyl-ether, 0,8 g | 65 | 94 |
| 31 | PEG 35000, 270 g | Vinylacetat, 410 g | N,N'-Divinyl-ethylenharnstoff 0,7 g | 73 | 95 |
| 32 | PEG 12000, 270 g | Vinylacetat, 410 g | Pentaerythrittriallylether, 1,6 g | 50 | 94 |

[1] PEG x: Polyethylenglykol mit mittlerem Molekulargewicht x

Beispiel 34: Umsetzung mit 3-Chlor-2-hydroxypropyl-trimethylammoniumchlorid

Zu 400 g einer 15,3%igen Lösung aus Beispiel 26 gibt man 46 g einer 60%igen wässerigen Lösung von 3-Chlor-2-hydroxypropyltrimethylammoniumchlorid sowie 6 g Natriumhydroxid. Man rührt 3 Stunden bei 60°C und anschließend zwei weitere Stunden bei 90°C. Man erhält eine klare Lösung.

B Anwendungsbeispiele

Anwendungsbeispiel 2: Duschgel

Gemäß folgender Rezeptur wurde zunächst eine erfindungsgemäße Duschgel-Formulierung (Duschgel A) hergestellt:

| Zusatz | | Gew.-% |
|---|---|---|
| Texapon NSO | Natriumlaurethsulfat | 40,0 |
| Tego Betain L7 | Cocamidopropylbetain | 5,0 |
| Plantacare 2000 | Decylglucosid | 5,0 |
| Parfüm | | 0,2 |
| Polymer gemäß Herstellungsbeispiel 25 | | 0,2 |
| Euxyl K 100 | Benzylalkohol, Methylchlorisothiazolinon, Methylisothiazolinon | 0,1 |
| D-Panthenol USP | | 0,5 |
| Citronensäure (pH 6-7) | | q.s. |
| NaCl | | 2,0 |
| Wasser | | ad 100 |

In gleicher Weise wurden drei Vergleichs-Duschgele hergestellt und zwar:

Duschgel B: (erfindungsgemäßes Copolymer ersetzt durch gleiche Menge kationisch modifizierter Hydroxyethylcellulose)

Duschgel C: (ohne Polymerzusatz)

Mit den Duschgelen A, B, und C wurde der folgende Vergleichs-test 3 zur Bestimmung der Schaumcremigkeit durchgeführt:

Je 2,0 g der oben genannten Formulierung wurde auf die linke Handinnenfläche gegeben, mit Leitungswasser angeschäumt und nach 1 Minute Reiben zwischen beiden Händen das Schaumgefühl in den Handinnenflächen beurteilt:

Note 1: sehr cremig/sahnig
Note 2: cremig/sahnig
Note 3: stumpf/gehaltlos

Ergebnis von Vergleichstest 3 (Mittelwert der Benotung durch 10 Probanden):

| Duschgel | Mittelwert von 10 Probanden |
|---|---|
| A | 1,3 |
| B | 2,1 |
| C | 2,8 |

Anwendungsbeispiel 7: Hydrogel zur Hautpflege

| Zusatz | Gew.-% |
|---|---|
| Polymer (Herstellungsbeispiel 29) | 3,0 |
| Sorbit | 2,0 |

(fortgesetzt)

| Zusatz | Gew.-% |
|---|---|
| Glycerin | 3,0 |
| Polyethylenglycol 400 | 5,0 |
| Ethanol | 1,0 |
| Parfümöl | q.s. |
| Konservierungsmittel | q.s. |
| Wasser | ad 100 |

Anwendungsbeispiel 8: Hydrodispersionsgel

| Zusatz | Gew.-% |
|---|---|
| Polymer (Herstellungsbeispiel 26) | 3,0 |
| Sorbit | 2,0 |
| Glycerin | 3,0 |
| Polyethylenglycol 400 | 5,0 |
| Triglycerid, flüssig | 2,0 |
| Ethanol | 1,0 |
| Parfümöl | q.s. |
| Konservierungsmittel | q.s. |
| Wasser | ad 100 |

Anwendungsbeispiel 12 Schimmerndes Gel

A

32,6 Dest. Wasser
0,1 Disodium EDTA
25,0 Carbomer (2%ige wässrige Lösung)
0,3 Konservierungsmittel

B

0,5 Dest. Wasser
0,5 Triethanolamine

C

10,0 Dest. Wasser
9,0 Polymerisat gemäß Herstellbeispiel 31
1,0 Polyquaternium-46

5,0 Iron Oxide

D

15, 0 Dest. Wasser
1,0 D-Panthenol 50 P (Panthenol und Propylene Glycol)

Herstellung:

Mit einem Propellermischer die Einsatzstoffe von Phase A in der angegebenen Reihenfolge gut mischen. Dann Phase B in Phase A hineingeben. Langsam rühren bis alles homogen ist. Phase C gut homogenisieren, bis die Pigmente gut verteilt sind. Phase C und Phase D zu Phase AB geben und gut mischen.

Anwendungsbeispiel 16 Sun Protection Lotion

| | |
|---|---|
| Phase A | |
| 6,00 | Octyl Methoxycinnamate (Uvinul MC 80 ™ von BASF) |
| 2,50 | 4-Methylbenzylidene Camphor (Uvinul MBC 95 ™ von BASF) |
| 1,00 | Octyl Triazone (Uvinul T 150 ™ von BASF) |
| 2,00 | Butyl Methoxydibenzoylmethane (Uvinul BMBM ™ von BASF) |
| 2,00 | PVP/Hexadecene Copolymer |
| 5,00 | PPG-3 Myristyl Ether |
| 0,50 | Dimethicone |
| 0,10 | BHT, Ascorbyl Palmitate, Citric Acid, Glyceryl Stearate, Propylene Glycol |
| 2,00 | Cetyl Alcohol |
| 2,00 | Potassium Cetyl Phosphate |
| Phase B | |
| 2,50 | Polymerisat gemäß Herstellbeispiel 25 |
| 5,00 | Propylene Glycol |
| 0,20 | Disodium EDTA |
| q.s. | Konservierungsmittel |
| 63,92 | dest. Wasser |
| Phase C | |
| 5,00 | Mineral Oil |
| 0,20 | Carbomer |
| Phase D | |
| 0,08 | Sodium Hydroxide |
| Phase E | |
| q.s. | Parfümöl |

Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

## Patentansprüche

**1.** Verwendung von Polymerisaten, die erhältlich sind durch radikalische Polymerisation von

a) mindestens einem Vinylester von $C_1$-$C_{24}$-Carbonsäuren in Gegenwart von

b) polyetherhaltigen Verbindungen mit einem Molekulargewicht von mindestens 300, und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff,

in hautkosmetischen Formulierungen und in der dekorativen Kosmetik.

**2.** Verwendung nach Anspruch 1, **dadurch gekennzeichnet, daß** im Anschluß an die Polymerisation die Esterfunktionen der ursprünglichen Monomeren a) zumindest teilweise verseift werden.

**3.** Verwendung nach Anspruch 1 und 2, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Verbindungen der allgemeinen Formel I

$$R^1\left(O-(R^2-O)_u-(R^3-O)_v-(R^4-O)_w\left[A-R^2-O)_x-(R^3-O)_y-R^4-O)_z\right]_s-R^5\right)_n$$

(I)

in der die Variablen unabhängig voneinander folgende Bedeutung haben:

| | |
|---|---|
| $R^1$ | Wasserstoff, $C_1$-$C_{24}$-Alkyl, $R^6$-C(=O)-, $R^6$-NH-C(=O)-, Polyalkoholrest; |
| $R^5$ | Wasserstoff, $C_1$-$C_{24}$-Alkyl, $R^6$-C(=O)-, $R^6$-NH-C(=O)-; |
| $R^2$ bis $R^4$ | - $(CH_2)_2$-, -$(CH_2)_3$-, -$(CH_2)_4$-, -$CH_2$-CH($R^6$)-,<br>- $CH_2$-CHO$R^7$-$CH_2$-; |
| $R^6$ | $C_1$-$C_{24}$-Alkyl; |
| $R^7$ | Wasserstoff, $C_1$-$C_{24}$-Alkyl, $R^6$-C(=O)-, $R^6$-NH-C(=O)-; |
| A | -C(=O)-O, -C(=O)-B-C(=O)-O,<br>- C(=O)-NH-B-NH-C(=O)-O; |
| B | -$(CH_2)_t$-, Arylen, ggf. substituiert; |
| n | 1 bis 1000; |
| s | 0 bis 1000; |
| t | 1 bis 12; |
| u | 1 bis 5000; |
| v | 0 bis 5000; |

w 0 bis 5000;

x 0 bis 5000;

y 0 bis 5000;

z 0 bis 5000;

und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff.

**4.** Verwendung von Polymerisaten nach Anspruch 1, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Verbindungen der allgemeinen Formel I mit einem mittleren Molekulargewicht von 300 bis 100000 (nach dem Zahlenmittel), in der die Variablen unabhängig voneinander folgende Bedeutung haben:

$R^1$ Wasserstoff, $C_1$-$C_{12}$-Alkyl, $R^6$-C(=O)-, $R^6$-NH-C(=O)-, Polyalkoholrest;

$R^5$ Wasserstoff, $C_1$-$C_{12}$-Alkyl, $R^6$-C(=O)-, $R^6$-NH-C(=O)-;

$R^2$ bis $R^4$ - $(CH_2)_2$-, -$(CH_2)_3$-, -$(CH_2)_4$-, -$CH_2$-CH($R^6$)-,<br>- $CH_2$-CHOR$^7$-$CH_2$-;

$R^6$ $C_1$-$C_{12}$-Alkyl;

$R^7$ Wasserstoff, $C_1$-$C_{12}$-Alkyl, $R^6$-C(=O)-, $R^6$-NH-C(=O)-;

n 1 bis 8;

s 0;

u 2 bis 2000;

v 0 bis 2000;

w 0 bis 2000;

und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff.

**5.** Verwendung von Polymerisaten nach Anspruch 1, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Verbindungen der allgemeinen Formel I mit einem mittleren Molekulargewicht von 500 bis 50000 (nach dem Zahlenmittel), in der die Variablen unabhängig voneinander folgende Bedeutung haben:

$R^1$ Wasserstoff, $C_1$-$C_6$-Alkyl, $R^6$-C(=O)-, $R^6$-NH-C(=O)-;

| | |
|---|---|
| $R^5$ | Wasserstoff, $C_1$-$C_6$-Alkyl, $R^6$-C(=O)-, $R^6$-NH-C(=O)-; |
| $R^2$ bis $R^4$ | - $(CH_2)_2$-, -$(CH_2)_3$-, -$(CH_2)_4$-, -$CH_2$-CH($R^6$)-, - $CH_2$-CHOR$^7$-$CH_2$-; |
| $R^6$ | $C_1$-$C_6$-Alkyl; |
| $R^7$ | Wasserstoff, $C_1$-$C_6$-Alkyl, $R^6$-C(=O)-, $R^6$-NH-C(=O)-; |
| n | 1; |
| s | 0; |
| u | 5 bis 500; |
| v | 0 bis 500; |
| w | 0 bis 500; |

und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff,

in hautkosmetischen Formulierungen.

**6.** Verwendung von Polymerisaten nach Anspruch 1, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Silikonderivaten
und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff.

**7.** Verwendung von Polymerisaten nach Anspruch 6, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Silikonderivaten der allgemeinen Formel II

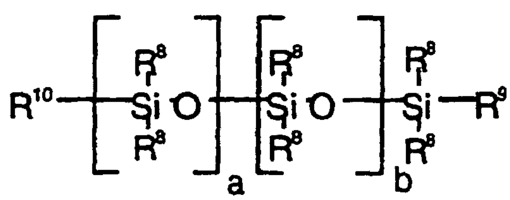

(II)

wobei:

$R^9 = CH_3$ oder 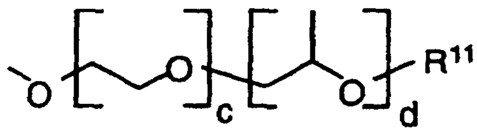

$R^{10} = CH_3$ oder $R^9$ 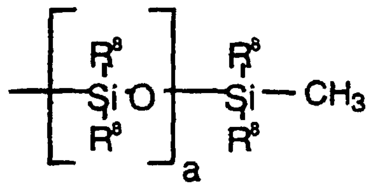

$R^{11} = H, CH_3,$

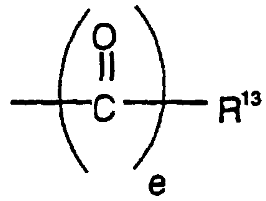

$R^{13}$ ein organischer Rest aus 1 bis 40 Kohlenstoffatomen, der Amino-, Carbonsäure- oder Sulfonatgruppen enthalten kann, oder für den Fall e=0, auch das Anion einer anorganischen Säure bedeutet,

und wobei die Reste $R^8$ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich $R^{12}$ sind, wobei:

$R^{12} =$ 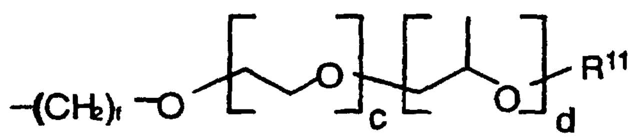

mit der Maßgabe, daß mindestens einer der Reste $R^8$, $R^9$ oder $R^{10}$ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist, und e 0 oder 1 ist,
und
einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff.

8. Verwendung von Polymerisaten nach Anspruch 7, **dadurch gekennzeichnet, daß** Formel II folgende Bedeutung besitzt:

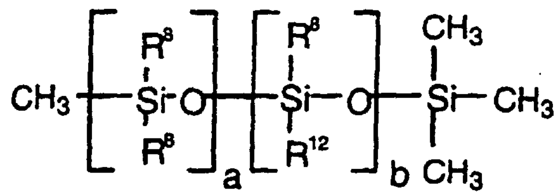

**9.** Verwendung nach Anspruch 1, **dadurch gekennzeichnet, daß** die Polymerisate erhältlich sind durch radikalische Polymerisation von

a) mindestens einem Vinylester von $C_1$-$C_{24}$-Carbonsäuren in Gegenwart von

b) polyetherhaltigen Verbindungen erhältlich durch Umsetzung von Polyethyleniminen mit Alkylenoxiden und

c) einem oder mehreren weiteren copolymerisierbaren Monomeren, ausgewählt aus der Gruppe der Monomere Methylmethacrylat, N-Vinylpyrrolidon, 3-Methyl-1-vinyl-imidazoliummethylsulfat, N-Vinylformamid, Pentaerythrit-triallylether und N,N'-Divinyl-ethylenharnstoff.

**10.** Verwendung von Polymerisaten nach Anspruch 9, **dadurch gekennzeichnet, daß** als Alkylenoxide Ethylenoxid, Propylenoxid, Butylenoxid und Mischungen aus diesen verwendet werden.

**11.** Verwendung von Polymerisaten nach Ansprüchen 9 und 10, **dadurch gekennzeichnet, daß** als Alkylenoxid Ethylenoxid verwendet wird.

**12.** Verwendung von Polymerisaten nach Ansprüchen 9, 10 und 11, **dadurch gekennzeichnet, daß** das Polyethylenimin ein Molekulargewicht zwischen 300 und 20000 besitzt.

**13.** Verwendung von Polymerisaten nach Anspruch 1, **dadurch gekennzeichnet, daß** die polyetherhaltigen Verbindungen b) durch Polymerisation von ethylenisch ungesättigten alkylenoxidhaltigen Monomeren und gegebenenfalls weiteren copolymerisierbaren Monomeren hergestellt worden sind.

**14.** Verwendung von Polymerisaten nach Anspruch 13, **dadurch gekennzeichnet, daß** die polyetherhaltigen Verbindungen b) durch Polymerisation von Polyalkylenoxidvinylethern und gegebenenfalls weiteren copolymerisierbaren Monomeren hergestellt worden sind.

**15.** Verwendung von Polymerisaten nach Anspruch 13, **dadurch gekennzeichnet, daß** die polyetherhaltigen Verbindungen b) durch Polymerisation von Polyalkylenoxid(meth)acrylaten und gegebenenfalls weiteren copolymerisierbaren Monomeren hergestellt worden sind.

**16.** Verwendung von Polymerisaten nach Anspruch 1, **dadurch gekennzeichnet, daß** die Mengenverhältnisse

a) 10 - 98 Gew.-%
b) 2 - 90 Gew.-%
c) bis 50 Gew.-%

betragen.

**17.** Verwendung von Polymerisaten nach Ansprüchen 1 bis 15, **dadurch gekennzeichnet, daß** die Mengenverhältnisse

a) 50 - 97 Gew.-%
b) 3 - 50 Gew.-%
c) bis 30 Gew.-%

betragen.

**18.** Verwendung von Polymerisaten nach Ansprüchen 1 bis 15, **dadurch gekennzeichnet, daß** die Mengenverhältnisse

a) 65 - 97 Gew.-%
b) 3 - 35 Gew.-%
c) bis 20 Gew.-%
betragen.

**19.** Hautkosmetische Formulierungen, **dadurch gekennzeichnet, daß** sie wie folgt zusammengesetzt sind:

| | | |
|---|---|---|
| a) | 0,05 - 20 Gew.-% | des Polymeren gemäß Anspruch 1 |
| b) | 20 - 99,95 Gew.-% | Wasser und/oder Lösungsmittel und/oder einer Ölkomponente |
| c) | 0 - 79,5 Gew.-% | weitere Bestandteile |

**20.** Hautkosmetische Formulierung gemäß Anspruch 19, **dadurch gekennzeichnet, daß** es sich um eine Emulsion handelt, die wie folgt zusammengesetzt ist:

| | | |
|---|---|---|
| a) | 0,05 - 10 Gew.-% | des Polymeren gemäß Anspruch 1 |
| b) | 10 - 94,94 Gew.-% | Wasser |
| c) | 5 - 89,94 Gew.-% | einer Ölkomponente |
| d) | 0,01 - 40 Gew.-% | eines Emulgators |
| e) | 0 - 74,94 Gew.-% | weitere Bestandteile |

**21.** Hautkosmetische Formulierung gemäß Ansprüchen 19 und 20, **dadurch gekennzeichnet, daß** es sich um eine W/O-Emulsion handelt, die wie folgt zusammengesetzt ist:

| | | |
|---|---|---|
| a) | 0,05 - 10 Gew.-% | des Polymeren gemäß Anspruch 1 |
| b) | 20 - 77,95 Gew.-% | Wasser |
| c) | 20 - 77,95 Gew.-% | einer Ölkomponente |
| d) | 2 - 35 Gew.-% | eines Emulgators |
| e) | 0 - 55,95 Gew.-% | weitere Bestandteile |

**22.** Hautkosmetische Formulierung gemäß Ansprüchen 19 und 20, **dadurch gekennzeichnet, daß** es sich um eine O/W-Emulsion handelt, die wie folgt zusammengesetzt ist:

| | | |
|---|---|---|
| a) | 0,05 - 10 Gew.-% | des Polymeren gemäß Anspruch 1 |
| b) | 40 - 96,95 Gew.-% | Wasser |
| c) | 1 - 44,95 Gew.-% | einer Ölkomponente |
| d) | 1 - 35 Gew.-% | eines Emulgators |
| e) | 0 - 10 Gew.-% | eines Gelbildners |
| f) | 0 - 57,95 Gew.-% | weitere Bestandteile |

## Claims

**1.** The use of polymers obtainable by free-radical polymerization of

a) at least one vinyl ester of $C_1$-$C_{24}$-carboxylic acids in the presence of

b) polyether-containing compounds having a molecular weight of at least 300, and

c) one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea,

in skin cosmetic formulations and in decorative cosmetics.

**2.** The use as claimed in claim 1, wherein, after the polymerization, the ester functions of the original monomers a) are at least partially hydrolyzed.

**3.** The use as claimed in claim 1 and 2, wherein the polymers are obtainable by free-radical polymerization of

a) at least one vinyl ester of $C_1$-$C_{24}$-carboxylic acids in the presence of

b) polyether-containing compounds of the formula I

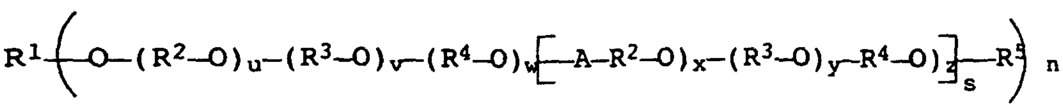

(I)

in which the variables independently of one another have the following meanings:

| | |
|---|---|
| $R^1$ | is hydrogen, $C_1$-$C_{24}$-alkyl, $R^6$-C(=O)-, $R^6$-NH-C(=O)-, polyalcohol radical; |
| $R^5$ | is hydrogen, $C_1$-$C_{24}$-alkyl, $R^6$-C(=O)-, $R^6$-NH-C(=O)-; |
| $R^2$ to $R^4$ | are<br>- $(CH_2)_2$-, -$(CH_2)_3$-, -$(CH_2)_4$-, -$CH_2$-CH($R^6$)-,<br>- $CH_2$-CHO$R^7$-$CH_2$-; |
| $R^6$ | is $C_1$-$C_{24}$-alkyl; |
| $R^7$ | is hydrogen, $C_1$-$C_{24}$-alkyl,- $R^6$-C(=O)-, $R^6$-NH-C(=O)-; |
| A | is -C(=O)-O, -C(=O)-B-C(=O)-O,<br>-C(=O)-NH-B-NH-C(=O)-O; |
| B | is -$(CH_2)_t$-, arylene, optionally substituted; |
| n | is 1 to 1 000; |
| s | is 0 to 1 000; |
| t | is 1 to 12; |
| u | is 1 to 5 000; |
| v | is 0 to 5 000; |
| w | is 0 to 5 000; |
| x | is 0 to 5 000; |
| y | is 0 to 5 000; |
| z | is 0 to 5 000; |

and

c) one or more further copolymerizable monomers chosen from the group of monomers consisting of methyl

methacrylate, N-vinylpyrrolidone, 3-methyl-1-vinylimidazolium methylsulfate, N-vinylformamide, pentaerythritol triallyl ether and N,N'-divinylethyleneurea.

**4.** The use of polymers as claimed in claim 1, wherein the polymers are obtainable by free-radical polymerization of

a) at least one vinyl ester of $C_1$-$C_{24}$-carboxylic acids in the presence of

b) polyether-containing compounds of the formula I having an average molecular weight of from 300 to 100 000 (according to the number average), in which the variables independently of one another have the following meanings:

$R^1$ is hydrogen, $C_1$-$C_{12}$-alkyl, $R^6$-C(=O)-, $R^6$-NH-C(=O)-, polyalcohol radical;

$R^5$ is hydrogen, $C_1$-$C_{12}$-alkyl, $R^6$-C(=O)-, $R^6$-NH-C(=O)-;

$R^2$ to $R^4$ are
$-(CH_2)_2-$, $-(CH_2)_3-$, $-(CH_2)_4-$, $-CH_2-CH(R^6)-$,
$- CH_2-CHOR^7-CH_2-$;

$R^6$ is $C_1$-$C_{12}$-alkyl;

$R^7$ is hydrogen, $C_1$-$C_{12}$-alkyl, $R^6$-C(=O)-, $R^6$-NH-C(=O)-;

n is 1 to 8;

s is 0;

u is 2 to 2 000;

v is 0 to 2 000;

w is 0 to 2 000;

and

c) one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea.

**5.** The use of polymers as claimed in claim 1, wherein the polymers are obtainable by free-radical polymerization of

a) at least one vinyl ester of $C_1$-$C_{24}$-carboxylic acids in the presence of

b) polyether-containing compounds of the formula I having an average molecular weight of from 500 to 50 000 (according to the number average), in which the variables independently of one another have the following meaning:

$R^1$ is hydrogen, $C_1$-$C_6$-alkyl, $R^6$-C(=O)-, $R^6$-NH-C(=O)-;

$R^5$ is hydrogen, $C_1$-$C_6$-alkyl, $R^6$-C(=O)-, $R^6$-NH-C(=O)-;

$R^2$ to $R^4$ are
$-(CH_2)_2-$, $-(CH_2)_3-$, $-(CH_2)_4-$, $-CH_2-CH(R^6)-$,
$- CH_2-CHOR^7-CH_2-$;

$R^6$ is $C_1$-$C_6$-alkyl;

$R^7$ is hydrogen, $C_1$-$C_6$-alkyl, $R^6$-C(=O)-, $R^6$-NH-C(=O)-;

n is 1;

s is 0;

u is 5 to 500;

v is 0 to 500;

w is 0 to 500;

and

c) one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea,

in skin cosmetic formulations.

**6.** The use of polymers as claimed in claim 1, wherein the polymers are obtainable by free-radical polymerization of

a) at least one vinyl ester of $C_1$-$C_{24}$-carboxylic acids in the presence of

b) polyether-containing silicone derivatives
and

c) one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea.

**7.** The use of polymers as claimed in claim 6, wherein the polymers are obtainable by free-radical polymerization of

a) at least one vinyl ester of $C_1$-$C_{24}$-carboxylic acids in the presence of

b) polyether-containing silicone derivatives of the formula II

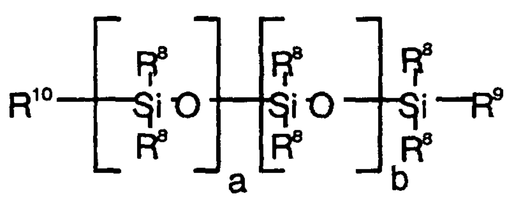

(II)

where:

$R^9 = CH_3$ or 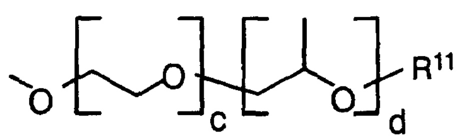

$R^{10} = CH_3$ or $R^9$

$R^{11} = H, CH_3,$

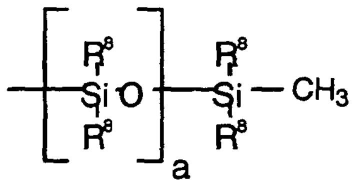

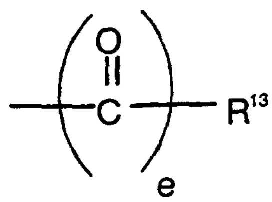

$R^{13}$ is a $C_1$-$C_{40}$ organic radical which can contain amino, carboxylic acid or sulfonate groups, or for the case e = 0, is also the anion of an inorganic acid,

and where the radicals $R^8$ can be identical or different, and either originate from the group of aliphatic hydrocarbons having 1 to 20 carbon atoms, are cyclic aliphatic hydrocarbons having 3 to 20 carbon atoms, are of an aromatic nature or are identical to $R^{12}$, where:

$R^{12} =$ 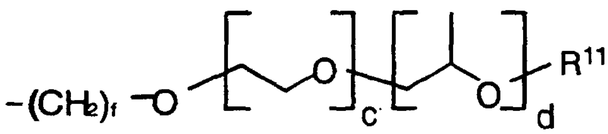

with the proviso that at least one of the radicals $R^8$, $R^9$ or $R^{10}$ is a polyalkylene oxide-containing radical as defined above,
and f is an integer from 1 to 6,
a and b are integers such that the molecular weight of the polysiloxane block is between 300 and 30 000,
c and d can be integers between 0 and 50, with the proviso that the sum c + d is greater than 0, and e is 0 or 1, and
one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea.

**8.** The use of polymers as claimed in claim 7, wherein formula II has the following meaning:

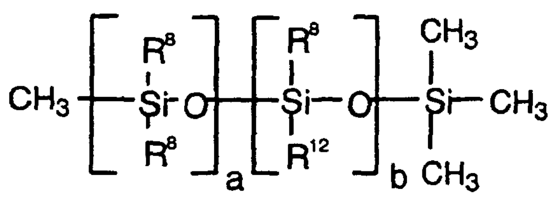

**9.** The use as claimed in claim 1, wherein the polymers are obtainable by free-radical polymerization of

a) at least one vinyl ester of $C_1$-$C_{24}$-carboxylic acids in the presence of

b) polyether-containing compounds obtainable by reaction of polyethyleneimines with alkylene oxides and

c) one or more further copolymerizable monomers chosen from the group of monomers consisting of methyl methacrylate, N-vinylpyrrolidone, 3-methyl-1-vinylimidazolium methylsulfate, N-vinylformamide, pentaerythritol triallyl ether and N,N'-divinylethyleneurea.

**10.** The use of polymers as claimed in claim 9, wherein the alkylene oxides are ethylene oxide, propylene oxide, butylene oxide and mixtures thereof.

**11.** The use of polymers as claimed in claims 9 and 10, wherein the alkylene oxide is ethylene oxide.

**12.** The use of polymers as claimed in claims 9, 10 and 11, wherein the polyethyleneimine has a molecular weight between 300 and 20 000.

**13.** The use of polymers as claimed in claim 1, wherein the polyether-containing compounds b) have been prepared by polymerization of ethylenically unsaturated alkylene oxide-containing monomers and optionally further copolymerizable monomers.

**14.** The use of polymers as claimed in claim 13, wherein the polyether-containing compounds b) have been prepared by polymerization of polyalkylene oxide vinyl ethers and optionally further copolymerizable monomers.

**15.** The use of polymers as claimed in claim 13, wherein the polyether-containing compounds b) have been prepared by polymerization of polyalkylene oxide (meth)acrylates and optionally further copolymerizable monomers.

**16.** The use of polymers as claimed in claim 1, wherein the quantitative ratios are

a) 10 - 98% by weight
b) 2 - 90% by weight
c) up to 50% by weight.

**17.** The use of polymers as claimed in claims 1 to 15, wherein the quantitative ratios are

a) 50 - 97% by weight
b) 3 - 50% by weight
c) up to 30% by weight.

**18.** The use of polymers as claimed in claims 1 to 15, wherein the quantitative ratios are

a) 65 - 97% by weight
b) 3 - 35% by weight
c) up to 20% by weight.

**19.** A skin cosmetic formulation which has the following composition:

| | | |
|---|---|---|
| a) | 0.05 - 20% | by weight of the polymer as in claim 1 |
| b) | 20 - 99.95% | by weight of water and/or solvents and/or an oil component |
| c) | 0 - 79.5% | by weight of further constituents. |

**20.** A skin cosmetic formulation as claimed in claim 19 which is an emulsion and has the following composition:

| | | |
|---|---|---|
| a) | 0.05 - 10% | by weight of the polymer as in claim 1 |
| b) | 10 - 94.94% | by weight of water |
| c) | 5 - 89.94% | by weight of an oil component |
| d) | 0.01 - 40% | by weight of an emulsifier |
| e) | 0 - 74.94% | by weight of further constituents. |

**21.** A skin cosmetic formulation as claimed in claims 19 and 20 which is a W/O emulsion and has the following composition:

| | | |
|---|---|---|
| a) | 0.05 - 10% | by weight of the polymer as in claim 1 |
| b) | 20 - 77.95% | by weight of water |
| c) | 20 - 77.95% | by weight of an oil component |
| d) | 2 - 35% | by weight of an emulsifier |
| e) | 0 - 55.95% | by weight of further constituents. |

**22.** A skin cosmetic formulation as claimed in claims 19 and 20 which is an O/W emulsion and has the following composition:

| | | |
|---|---|---|
| a) | 0.05 - 10% | by weight of the polymer as in claim 1 |
| b) | 40 - 96.95% | by weight of water |
| c) | 1 - 44.95% | by weight of an oil component |
| d) | 1 - 35% | by weight of an emulsifier |
| e) | 0 - 10% | by weight of a gel former |
| f) | 0 - 57.95% | by weight of further constituents. |

## Revendications

**1.** Utilisation de produits de polymérisation, qui peuvent être obtenus par polymérisation radicalaire de

a) au moins un ester vinylique d'acides carboxyliques en $C_1$-$C_{24}$ en présence de

b) composés contenant des polyéthers, ayant une masse moléculaire d'au moins 300, et

c) un ou plusieurs autres monomères copolymérisables, choisis dans le groupe des monomères méthacrylate de méthyle, N-vinylpyrrolidone, méthylsufate de 3-méthyl-1-vinyl-imidazolinium, N-vinylformamide, éther triallylique de pentaérythrite et N,N'-divinyl-éthylèneurée,

dans des formulations cosmétiques pour la peau et en cosmétique décorative.

**2.** Utilisation selon la revendication 1, **caractérisée par le fait qu'**à la suite de la polymérisation les fonctions ester des monomères d'origine a) sont au moins partiellement saponifiées.

**3.** Utilisation selon la revendication 1 ou 2, **caractérisée par le fait que** les produits de polymérisation peuvent être obtenus par polymérisation radicalaire de

a) au moins un ester vinylique d'acides carboxyliques en $C_1$-$C_{24}$ en présence de

b) composés contenant des polyéthers de formule générale I

$$R^1\left(-O-(R^2-O)_u-(R^3-O)_v-(R^4-O)_w\left[-A-R^2-O)_x-(R^3-O)_y-R^4-O)_z\right]_s-R^5\right)_n$$

(I)

dans laquelle les variables ont indépendamment l'une de l'autre la signification suivante:

$R^1$ hydrogène, alkyle en $C_1$-$C_{24}$, $R^6$-C(=O)-, $R^6$-NH-C(=O)-, reste de polyalcool;

$R^5$ hydrogène, alkyle en $C_1$-$C_{24}$, $R^6$-C(=O)-, $R^6$-NH-C(=O)-;

$R^2$ à $R^4$ $-(CH_2)_2-$, $-(CH_2)_3-$, $-(CH_2)_4-$, $-CH_2CH(R^6)-$, $- CH_2-CHOR^7-CH_2-$;

$R^6$ alkyle en $C_1$-$C_{24}$;

$R^7$ hydrogène, alkyle en $C_1$-$C_{24}$, $R^6$-C(=O)-, $R^6$-NH-C(=O)-;

A -C(=O)-O, -C(=O)-B-C(=O)-O, -C(=O)-NH-B-NH-C(=O)-O;

B $-(CH_2)_t-$, arylène, éventuellement substitué;

n 1 à 1000;

s 0 à 1000;

t 1 à 12;

u 1 à 5000;

v 0 à 5000;

w 0 à 5000;

x 0 à 5000;

y 0 à 5000;

z 0 à 5000;

et

c) un ou plusieurs autres monomères copolymérisables, choisis dans le groupe des monomères méthacrylate de méthyle, N-vinylpyrrolidone, méthylsufate de 3-méthyl-1-vinylimidazolinium, N-vinylformamide, éther triallylique de pentaérythrite et N,N'-divinyl-éthylèneurée.

**4.** Utilisation de produits de polymérisation selon la revendication 1, **caractérisée par le fait que** les produits de polymérisation peuvent être obtenus par polymérisation radicalaire de

a) au moins un ester vinylique d'acides carboxyliques en $C_1$-$C_{24}$ en présence de

b) composés contenant des polyéthers de formule générale I, ayant une masse moléculaire moyenne de 300 à 100000 (selon la moyenne en nombre) et dans laquelle les variables ont indépendamment l'une de l'autre la signification suivante:

$R^1$ hydrogène, alkyle en $C_1$-$C_{12}$, $R^6$-C(=O)-, $R^6$-NH-C(=O)-, reste de polyalcool;

$R^5$ hydrogène, alkyle en $C_1$-$C_{12}$, $R^6$-C(=O)-, $R^6$-NH-C(=O)-;

$R^2$ à $R^4$ $-(CH_2)_2-$, $-(CH_2)_3-$, $-(CH_2)_4-$, $-CH_2CH(R^6)-$, $- CH_2-CHOR^7-CH_2-$;

$R^6$ alkyle en $C_1$-$C_{12}$;

$R^7$ hydrogène, alkyle en $C_1$-$C_{12}$, $R^6$-C(=O)-, $R^6$-NH-C(=O)-;

n 1 à 8;

s 0;

u 2 à 2000;

v 0 à 2000;

w 0 à 2000;

et

c) un ou plusieurs autres monomères copolymérisables, choisis dans le groupe des monomères méthacrylate de méthyle, N-vinylpyrrolidone, méthylsufate de 3-méthyl-1-vinylimidazolinium, N-vinylformamide, éther triallylique de pentaérythrite et N,N'-divinyl-éthylèneurée.

**5.** Utilisation de produits de polymérisation selon la revendication 1, **caractérisée par le fait que** les produits de polymérisation peuvent être obtenus par polymérisation radicalaire de

a) au moins un ester vinylique d'acides carboxyliques en $C_1$-$C_{24}$ en présence de

b) composés contenant des polyéthers de formule générale I, ayant une masse moléculaire moyenne de 500 à 50000 (selon la moyenne en nombre) et dans laquelle les variables ont indépendamment l'une de l'autre la signification suivante:

$R^1$ hydrogène, alkyle en $C_1$-$C_6$, $R^6$-C(=O)-, $R^6$-NH-C(=O)-;

$R^5$ hydrogène, alkyle en $C_1$-$C_6$, $R^6$-C(=O)-, $R^6$-NH-C(=O)-;

$R^2$ à $R^4$ $-(CH_2)_2-$, $-(CH_2)_3-$, $-(CH_2)_4-$, $-CH_2CH(R^6)-$, $-CH_2-CHOR^7-CH_2-$;

$R^6$ alkyle en $C_1$-$C_6$;

$R^7$ hydrogène, alkyle en $C_1$-$C_6$, $R^6$-C(=O)-, $R^6$-NH-C(=O)-;

n 1;

s 0;

u 5 à 500;

v 0 à 500;

w 0 à 500;

et

c) un ou plusieurs autres monomères copolymérisables, choisis dans le groupe des monomères méthacrylate de méthyle, N-vinylpyrrolidone, méthylsufate de 3-méthyl-1-vinylimidazolinium, N-vinylformamide, éther triallylique de pentaérythrite et N,N'-divinyl-éthylèneurée,

dans des formulations cosmétiques pour la peau.

**6.** Utilisation de produits de polymérisation selon la revendication 1, **caractérisée par le fait que** les produits de polymérisation peuvent être obtenus par polymérisation radicalaire de

a) au moins un ester vinylique d'acides carboxyliques en $C_1$-$C_{24}$ en présence de

b) dérivés de silicone contenant des polyéthers, et

c) un ou plusieurs autres monomères copolymérisables, choisis dans le groupe des monomères méthacrylate de méthyle, N-vinylpyrrolidone, méthylsufate de 3-méthyl-1-vinylimidazolinium, N-vinylformamide, éther triallylique de pentaérythrite et N,N'-divinyl-éthylèneurée.

7. Utilisation de produits de polymérisation selon la revendication 6, **caractérisée par le fait que** les produits de polymérisation peuvent être obtenus par polymérisation radicalaire de

a) au moins un ester vinylique d'acides carboxyliques en $C_1$-$C_{24}$ en présence de

b) dérivés de silicone contenant des polyéthers, de formule générale II

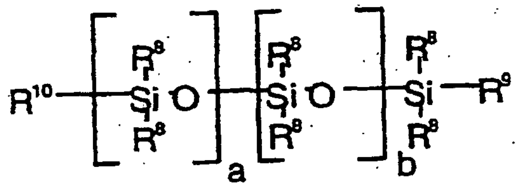

(II)

où:

$R^9 = CH_3$ ou

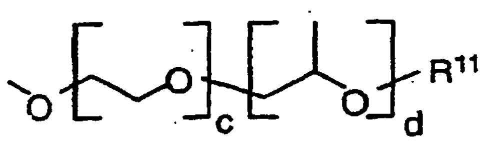

$R^{10} = CH_3$ ou $R^9$

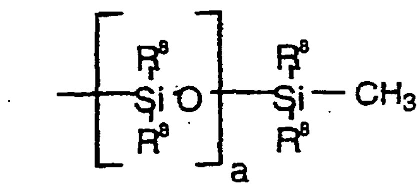

$R^{11}$ = H, $CH_3$,

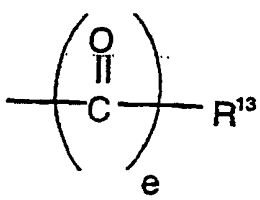

$R^{13}$ un reste organique ayant 1 à 40 atomes de carbone, qui peut contenir des groupes amino, acide carboxylique ou sulfonate, ou dans le cas où e = 0, désigne également l'anion d'un acide inorganique,

et tandis que les restes $R^8$ peuvent être identiques ou différents, et proviennent du groupe des hydrocarbures aliphatiques ayant 1 à 20 atomes de carbone, sont des hydrocarbures aliphatiques cycliques ayant 3 à 20 atomes de carbone, sont de nature aromatique ou sont identiques à $R^{12}$,

tandis que:

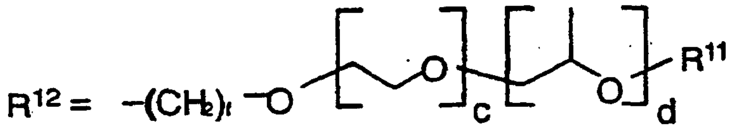

avec pour condition qu'au moins l'un des restes $R^8$, $R^9$ ou $R^{10}$ est un reste contenant du poly(oxyde d'alkylène) selon la définition indiquée plus haut,
et f est un nombre entier de 1 à 6,
a et b sont des nombres entiers tels que la masse moléculaire du bloc polysiloxane se situe entre 300 et 30000,
c et d peuvent être des nombres entiers entre 0 et 50, avec pour condition que la somme de c et d est supérieure à 0, et e est 0 ou 1,
et
un ou plusieurs autres monomères copolymérisables, choisis dans le groupe des monomères méthacrylate de méthyle, N-vinylpyrrolidone, méthylsufate de 3-méthyl-1-vinyl-imidazolinium, N-vinylformamide, éther triallylique de pentaérythrite et N,N'-divinyl-éthylèneurée.

**8.** Utilisation de produits de polymérisation selon la revendication 7, **caractérisée par le fait que** la formula II possède la signification suivante:

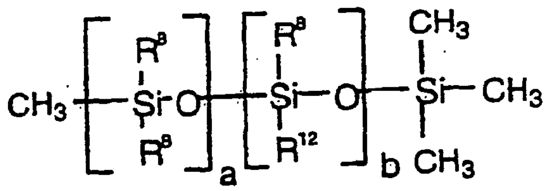

**9.** Utilisation selon la revendication 1, **caractérisée par le fait que** les produits de polymérisation peuvent être obtenus par polymérisation radicalaire de

a) au moins un ester vinylique d'acides carboxyliques en $C_1$-$C_{24}$ en présence de

b) composés contenant des polyéthers, pouvant être obtenus par réaction de polyéthylèneimines avec des oxydes d'alkylène
et

c) un ou plusieurs autres monomères copolymérisables, choisis dans le groupe des monomères méthacrylate de méthyle, N-vinylpyrrolidone, méthylsufate de 3-méthyl-1-vinylimidazolinium, N-vinylformamide, éther triallylique de pentaérythrite et N,N'-divinyl-éthylèneurée.

**10.** Utilisation de produits de polymérisation selon la revendication 9, **caractérisée par le fait qu'**on utilise comme oxydes d'alkylène de l'oxyde d'éthylène, de l'oxyde de propylène, de l'oxyde de butylène et des mélanges de ceux-ci.

**11.** Utilisation de produits de polymérisation selon les revendications 9 et 10, **caractérisée par le fait qu'**on utilise comme oxyde d'alkylène de l'oxyde d'éthylène.

**12.** Utilisation de produits de polymérisation selon les revendications 9, 10 et 11, **caractérisée par le fait que** la polyéthylèneimine possède une masse moléculaire entre 300 et 20000.

**13.** Utilisation de produits de polymérisation selon la revendication 1, **caractérisée par le fait que** les composés contenant des polyéther b) ont été préparés par polymérisation de monomères contenant de l'oxyde d'alkylène, éthyléniquement insaturés, et éventuellement d'autres monomères copolymérisables.

**14.** Utilisation de produits de polymérisation selon la revendication 13, **caractérisée par le fait que** les composés contenant des polyéther b) ont été préparés par polymérisation d'éthers vinyliques de poly(oxyde d'alkylène) et éventuellement d'autres monomères copolymérisables.

**15.** Utilisation de produits de polymérisation selon la revendication 13, **caractérisée par le fait que** les composés contenant des polyéther b) ont été préparés par polymérisation de (méth)acrylates de poly(oxyde d'alkylène) et éventuellement d'autres monomères copolymérisables.

**16.** Utilisation de produits de polymérisation selon la revendication 1, **caractérisée par le fait que** les proportions valent

a) 10 - 98 % en poids
b) 2 - 90 % en poids
c) jusqu'à 50 % en poids.

**17.** Utilisation de produits de polymérisation selon les revendications 1 à 15, **caractérisée par le fait que** les proportions valent

a) 50 - 97 % en poids
b) 3 - 50 % en poids
c) jusqu'à 30 % en poids.

**18.** Utilisation de produits de polymérisation selon les revendications 1 à 15, **caractérisée par le fait que** les proportions valent

a) 65 - 97 % en poids
b) 3 - 35 % en poids
c) jusqu'à 20 % en poids.

**19.** Formulations cosmétiques pour la peau, **caractérisées par le fait qu'**elles ont la composition suivante:

| | | |
|---|---|---|
| a) | 0,05 - 20 % en poids | du polymère selon la revendication 1 |
| b) | 20 - 99,95 % en poids | d'eau et/ ou de solvants et/ ou d'un composant de type huile |
| c) | 0 - 79,5 % en poids | d'autres constituants. |

**20.** Formulation cosmétique pour la peau selon la revendication 19, **caractérisée par le fait qu'**il s'agit d'une émulsion qui a la composition suivante:

| | | |
|---|---|---|
| a) | 0,05 - 10 % en poids | du polymère selon la revendication 1 |
| b) | 10 - 94,94 % en poids | d'eau |
| c) | 5 - 89,94 % en poids | d'un composant de type huile |
| d) | 0,01 - 40 % en poids | d'un émulsifiant |
| e) | 0 - 74,94 % en poids | d'autres constituants. |

**21.** Formulation cosmétique pour la peau selon les revendications 19 ou 20, **caractérisée par le fait qu'**il s'agit d'une émulsion E/H qui a la composition suivante:

| | | |
|---|---|---|
| a) | 0,05 - 10 % en poids | du polymère selon la revendication 1 |
| b) | 20 - 77,95 % en poids | d'eau |
| c) | 20 - 77,95 % en poids | d'un composant de type huile |
| d) | 2 - 35 % en poids | d'un émulsifiant |
| e) | 0 - 55,95 % en poids | d'autres constituants. |

**22.** Formulation cosmétique pour la peau selon les revendications 19 ou 20, **caractérisée par le fait qu'**il s'agit d'une émulsion H/E qui a la composition suivante:

| | | |
|---|---|---|
| a) | 0,05 - 10 % en poids | du polymère selon la revendication 1 |
| b) | 40 - 96,95 % en poids | d'eau |
| c) | 1 - 44,95 % en poids | d'un composant de type huile |
| d) | 1 - 35 % en poids | d'un émulsifiant |
| e) | 0 - 10 % en poids | d'un agent gélifiant |
| f) | 0 - 57,95 % en poids | d'autres constituants. |